# EUROPÄISCHE PATENTSCHRIFT

(11) **EP 1 590 672 B1**
(45) Veröffentlichungstag und Bekanntmachung des Hinweises auf die Patenterteilung: **08.10.2008**
(21) Anmeldenummer: 04706602.2
(22) Anmeldetag: 30.01.2004
(51) Int. Cl.: G01N 33/58, G01N 33/50

(54) **VERFAHREN ZUM NACHWEIS VON FERTILITÄTSSTEIGERNDEN ODER KONTRAZEPTIVEN WIRKSTOFFEN**
METHOD FOR THE DETECTION OF FERTILITY-BOOSTING OR CONTRACEPTIVE SUBSTANCES
PROCEDE DE DETECTION DE SUBSTANCES AUGMENTANT LA FERTILITE OU AYANT UN EFFET CONTRACEPTIF

(30) Priorität: 31.01.2003 DE 10303973
(43) Veröffentlichungstag der Anmeldung: 02.11.2005
(60) Teilanmeldung: 08160668.3
(73) Patentinhaber: Forschungszentrum Jülich GmbH, 52425 Jülich (DE)
(72) Erfinder: KAUPP, U., Benjamin, 52062 Aachen (DE); HILDEBRAND, Eilo, 52072 Aachen (DE); WEYAND, Ingbert, 52066 Aachen (DE)
(74) Vertreter: Dehmel, Albrecht
(86) Internationale Anmeldenummer: PCT/DE2004/000145
(87) Internationale Veröffentlichungsnummer: WO 2004/068142

(56) Entgegenhaltungen:
- WO-A-99/48902
- NISHIGAKI TAKUYA ET AL: "Time-resolved sperm responses to an egg peptide measured by stopped-flow fluorometry" BIOCHEMICAL AND BIOPHYSICAL RESEARCH COMMUNICATIONS, Bd. 284, Nr. 2, 8. Juni 2001 (2001-06-08), Seiten 531-535, XP002282306 ISSN: 0006-291X
- WIESNER B ET AL: "Measurement of intracellular Ca2+ changes using novel caged cyclic nucleotides and confocal laser scanning microscopy" JOURNAL OF PHOTOCHEMISTRY AND PHOTOBIOLOGY B BIOLOGY, Bd. 49, Nr. 2-3, April 1999 (1999-04), Seiten 112-119, XP002282307 ISSN: 1011-1344
- TATSU Y ET AL: "A caged sperm-activating peptide that has a photocleavable protecting group on the backbone amide" FEBS LETTERS, ELSEVIER SCIENCE PUBLISHERS, AMSTERDAM, NL, Bd. 525, Nr. 1-3, 14. August 2002 (2002-08-14), Seiten 20-24, XP004375887 ISSN: 0014-5793
- FURUTA T ET AL: "PHOTOCHEMICAL PROPERTIES OF NEW PHOTOLABILE CAMP DERIVATIVES IN A PHYSIOLOGICAL SALINE SOLUTION" JOURNAL OF ORGANIC CHEMISTRY, AMERICAN CHEMICAL SOCIETY. EASTON, US, Bd. 60, Nr. 13, 30. Juni 1995 (1995-06-30), Seiten 3953-3956, XP000611481 ISSN: 0022-3263
- WARD G E ET AL: "CHEMOTAXIS OF ARBACIA-PUNCTULATA SPERMATOZOA TO RESACT A PEPTIDE FROM THE EGG JELLY LAYER" JOURNAL OF CELL BIOLOGY, Bd. 101, Nr. 6, 1985, Seiten 2324-2329, XP002282308 ISSN: 0021-9525
- HO HAN-CHEN ET AL: "Hyperactivation of mammalian spermatozoa: Function and regulation" REPRODUCTION (CAMBRIDGE), Bd. 122, Nr. 4, Oktober 2001 (2001-10), Seiten 519-526, XP002282309 ISSN: 1470-1626
- KAUPP U BENJAMIN ET AL: "The signal flow and motor response controlling chemotaxis of sea urchin sperm." NATURE CELL BIOLOGY, Bd. 5, Nr. 2, Februar 2003 (2003-02), Seiten 109-117, XP001181602 ISSN: 1465-7392 (ISSN print)
- HAGEN: "Fluorescence spectroscopic quantification of the release of cyclic nucleotides from photocleavable [bis(carboxymethoxy)coumarin-4-yl]methyl esters inside cells." ANGEW. CHEM. INT ED., Bd. 41, Nr. 19, 4. Oktober 2002 (2002-10-04), Seiten 3625-3628,
- HAGEN: "high efficient and ultrafast phototriggers for cAMP and cGMP by using long-wavelength UV/Vis-activation" ANGEW. CHEM. INT ED., Bd. 40, Nr. 6, 2001, Seiten 1045-1048,
- KAUPP: "Cyclic nucleotide-gated ion channels." PHYSIOL REV, Bd. 82, 2002, Seiten 769-824,
- WIESNER: "Cyclic nucleotide-gated channels on the flagellum control Ca2+ entry into sperm." THE JOURNAL OF CELL BIOLOGY, Bd. 142, Nr. 2, 1998, Seiten 473-484,

## Beschreibung

Die vorliegende Veröffentlichung betrifft ein Verfahren zum Nachweis von Änderungen von zellulären Botenstoffen in Spermien in einer *Stopped-Flow*-Messvorrichtung oder zum Nachweis der Motilität oder der Änderung des Schwimmverhaltens in Spermien in einem Flüssigkeitsbehältnis. Die vorliegende Veröffentlichung betrifft ferner die Verwendung der Verfahren zur Identifizierung oder Charakterisierung von Wirkstoffen, die die Beweglichkeit und das Schwimmverhalten von Spermien beeinflussen.

Damit neues Leben entstehen kann, müssen sich Eizelle und Spermium treffen und ihre Kerne miteinander verschmelzen, d.h. das Spermium muss die Eizelle befruchten.

Die Eibefruchtung ist ein sehr komplexer Vorgang. Man kann sie in mehrere Stufen unterteilen, die hier schematisch dargestellt sind. Zunächst müssen die Spermien angelockt werden. Die Eizelle erreicht dies mit Hilfe von Lockstoffen, die sie an die Umgebung abgibt. Wenn ein Spermium derselben Spezies aufdie Schutzhülle der Eizelle trifft, wird es sehr spezifisch erkannt und festgehalten. Die Erkennung wird durch Proteine in der Eihülle und auf der Oberfläche des Spermiums vermittelt. In einem nächsten Schritt werden Proteine von dem Spennium ausgeschüttet, die die Eihülle verdauen und den Weg des Spermiums zur Zellmembran ebnen. Nachdem sich das Spermium "durchgefräst" hat, lagert es sich an die Zellmembran des Eies an; die Zellmembranen von Ei und Spermium verschmelzen miteinander. So gelangt der Zellkern des Spermiums in die Eizelle. Die verschiedenen Stadien der Eibefruchtung werden durch unbekannte chemische Faktoren kontrolliert. Diese Faktoren lösen im Spermium zelluläre Reaktionen aus, die das Bewegungsverhalten des Spermiums verändern.

Es ist sehr unwahrscheinlich, dass ein Spermium und eine Eizelle zufällig aufeinandertreffen. Um die Wahrscheinlichkeit eines Zusammentreffens zu erhöhen, werden sehr viele Spermien produziert und ausgeschüttet. In einem Milliliter menschlichen Spermas befinden sich bis zu 108 Spermien - also hundert Millionen. Von den rund 200 Millionen Spermien, die bei der Ejakulation in die Vagina ausgeschüttet werden, schaffen es nur ganz wenige - ca. 100 - 1000 Spermien - bis in die Nähe des Eies vorzudringen. Wie ihnen dies gelingt, ist weitgehend

Das Ei benutzt einen Trick, mit dem es die Spermien in seine unmittelbare Nähe lockt. Es schüttet einen Lockstoff aus, der den Spermien den Weg ins Ziel weist. Die Lockstoffe umhüllen die Eizelle mit einer Art chemischer Wolke. In unmittelbarer Nähe der Eizelle ist die Lockstoff-Konzentration hoch, und sie nimmt mit zunehmender Entfernung von der Eizelle ab, d.h. es entsteht ein Konzentrationsgefälle.

Die Spermien besitzen auf ihrer Oberfläche spezifische Rezeptoren; mit Hilfe dieser Sensoren können sie die Änderungen in der Lockstoff-Konzentration wahrnehmen. Aus dem Verhalten der Spermien lässt sich schließen, dass sie unterscheiden können, ob sie in einem Lockstoffgefälle bergauf oder bergab schwimmen - also zu der Quelle hin, oder von der Quelle wegschwimmen. Diese Fähigkeit ermöglicht den Spermien, sich in dem Konzentrationsgefälle des Lockstoffes zu orientieren. Die Fähigkeit, sich in einem chemischen Gradienten zu orientieren, nennt man Chemotaxis. Die Chemotaxis von Spermien ist vor allem in Seeigeln und Manteltieren untersucht worden, konnte aber auch in Säugetieren nachgewiesen werden.

Zur Zeit werden zur Empfängnisverhütung verschiedene Mittel, z.B. hormonelle oder chemische Kontrazeptiva, sowie Barrieremethoden (Präservativ, Okklusivpessar, Scheidendiaphragma) oder Sterilisation angewendet, die alle mehr oder weniger zuverlässig sind. Darüber hinaus greifen einige der Mittel - insbesondere die hormonellen Kontrazeptiva - in den allgemeinen Stoffwechsel der anwendenden Frau ein.

Andererseits werden teilweise enorme Anstrengungen unternommen, um den Kindenvunsch von Paaren zu erfüllen, die auf normalem Wege keine Kinder bekommen können. Die Ursachen für die Unfruchtbarkeit eines Mannes können beispielsweise in einer unterdurchschnittlichen Spermienzahl oder einer Unbeweglichkeit der Spermien liegen. Der Unfruchtbarkeit einer Frau können fehlende Lockstoffe ihrer Eizellen zugrunde liegen.

Daher liegt der Erfindung die Aufgabe zu Grunde, ein Verfahren bereitzustellen, mit dem einerseits endogene Substanzen und andererseits exogene oder synthetische Substanzen nachgewiesen, identifiziert und charakterisiert werden können, die die Motilität der Spermien fördern oder beeinträchtigen.

Die nachfolgenden Figuren erläutern die Erfindung.

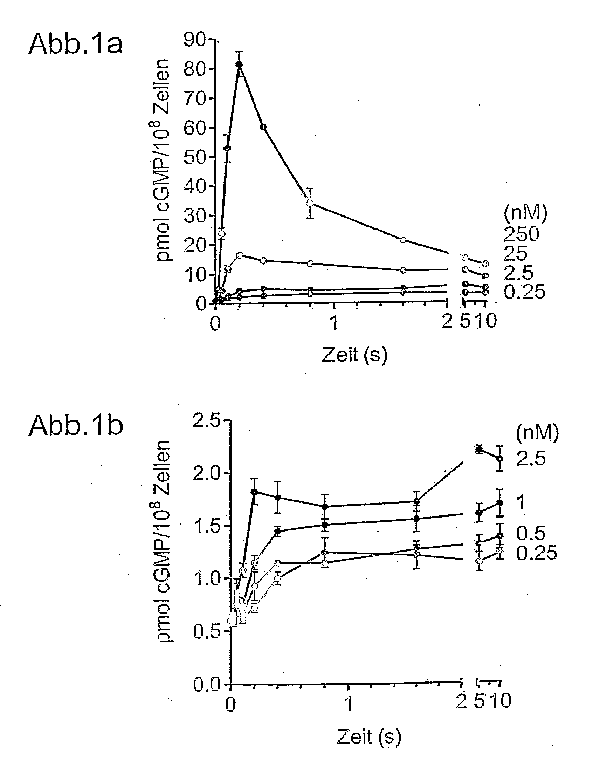

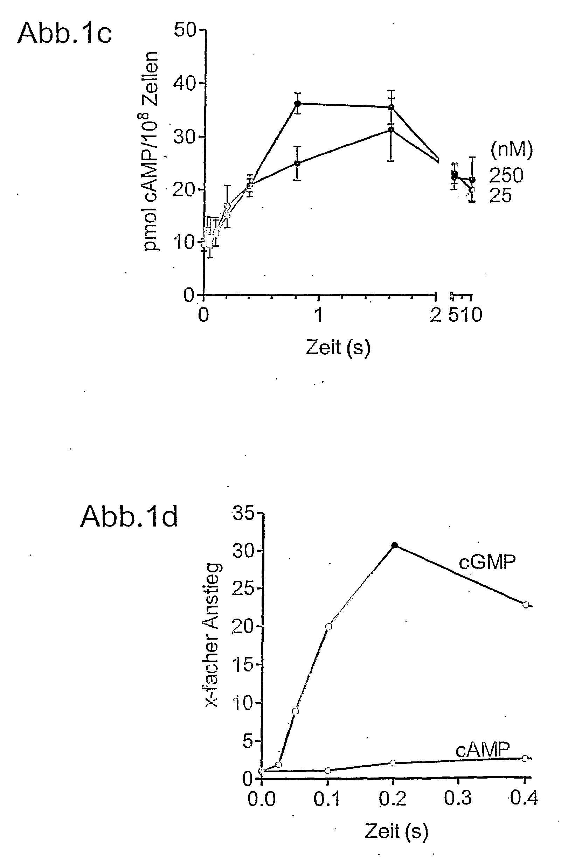

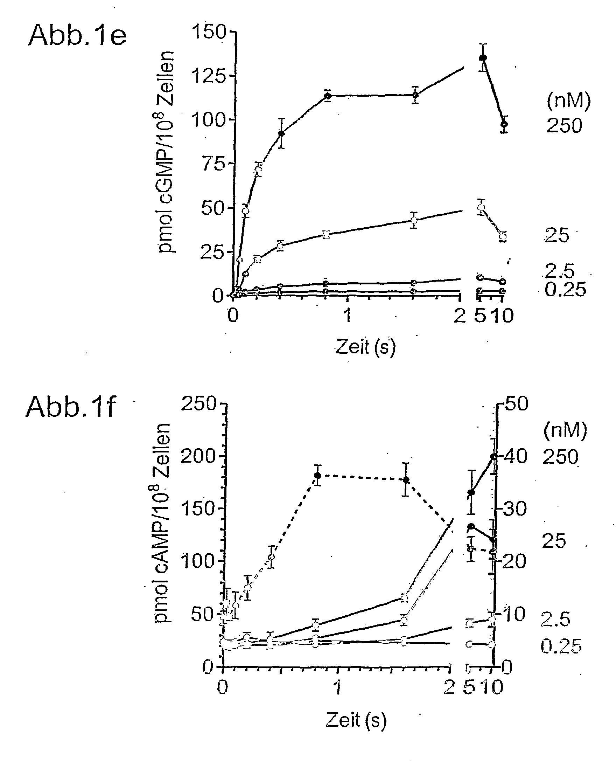

**Figur 1****:** Resact-induzierte Veränderungen der Konzentration zyklischer Nukleotide in Spermien. Die Daten stellen jeweils den Mittelwert aus drei Messungen eines Experiments dar. **a, b:** Veränderung der cGMP-Konzentration ([cGMP]) nach Stimulierung mit verschiedenen Resact-Konzentrationen. Die [cGMP] in unstimulierten Spermien ist bei t = 0 angegeben. Als Mittelwerte für den maximalen Anstieg wurden ermittelt: 30,6 ± 21,7 pMole/10⁸ Zellen (n = 9; 250 nM Resact); 8,7 ± 6,8 pMole/10⁸ Zellen (n = 6; 25 nM Resact); 3,3 ± 1,9 pMole/10⁸ Zellen (n = 6; 2,5 nM Resact); 1,9 ± 1,3 pMole/10⁸ Zellen (n = 3; 250 pM Resact). c: Veränderung der cAMP-Konzentration ([cAMP]) nach Stimulierung mit Resact. Als Mittelwerte für die maximalen Anstiege wurden ermittelt: 48,0 ± 30,5 pMole/10⁸ Zellen (n = 3; 250 nM Resact); 35,8 ± 18,6 pMole/10⁸ Zellen (n = 3; 25 nM Resact). **d:** Vergleich des Zeitverlaufes der relativen Veränderungen der [cGMP] und [cAMP] nach Stimulierung mit 250 nM Resact (Daten von **a** und **c**). **e:** Veränderung der [cGMP] nach Stimulierung mit verschiedenen Resact-Konzentrationen nach Vorinkubation der Spermien mit 2 mM IBMX (5 min). **f:** Veränderung der [cAMP] nach Stimulierung mit verschiedenen Resact-Konzentrationen nach Vorinkubation mit 2 mM IBMX (linke Ordinate). Die gepunktete Linie zeigt die Veränderung der [cAMP] in Spermien, die ohne IBMX mit 250 nM Resact stimuliert worden waren (rechte Ordinate).

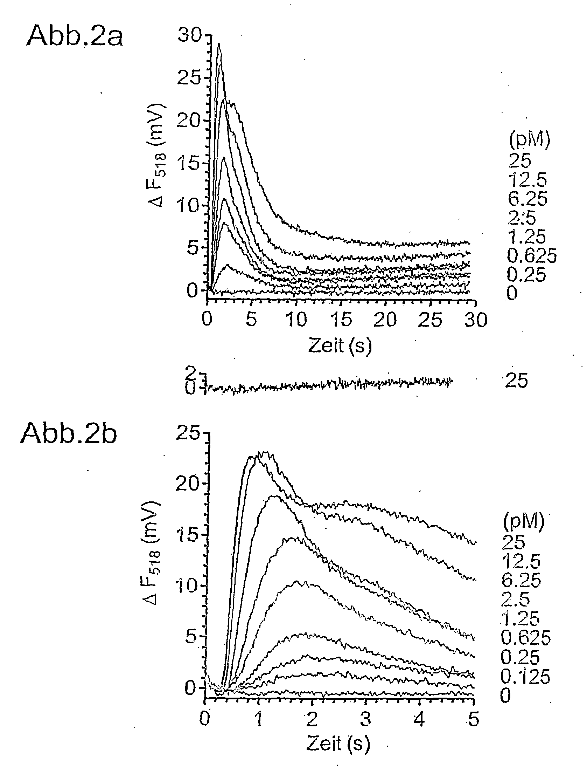

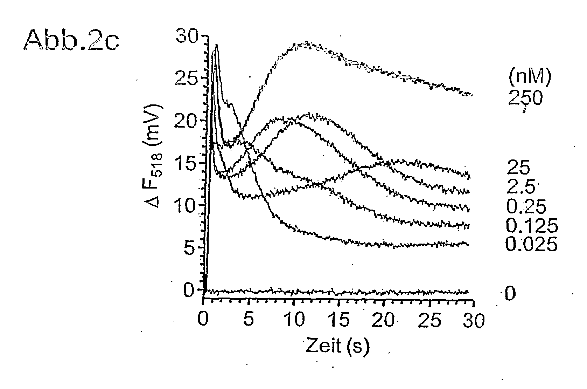

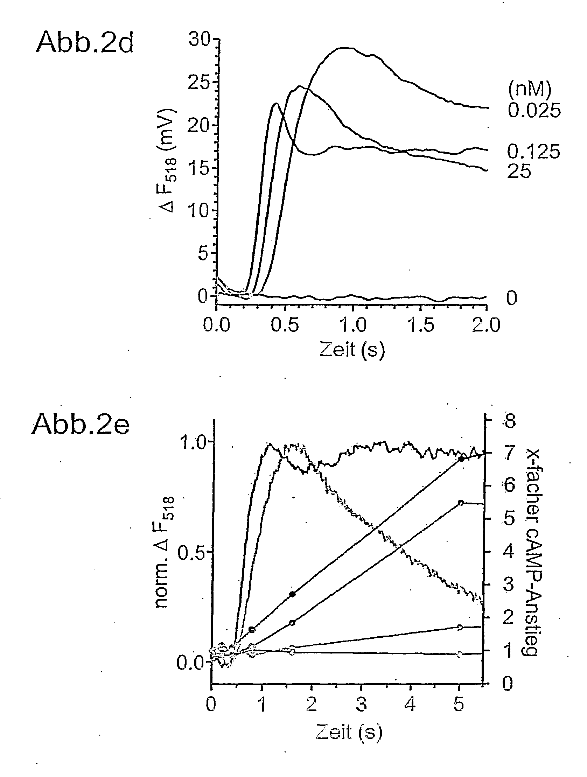

**Figur 2****:** Resact-induzierte Änderungen der intrazellulären Ca²⁺-Konzentration ([Ca²⁺]ᵢ) in Spermien. Die Änderungen wurden jeweils durch zeitaufgelöste Messungen der ΔF₅₁₈ von Fluo-4 bestimmt. **a:** Änderungen der [Ca²⁺]ᵢ. Die Spermien wurden bei t = 0 mit verschiedenen Resact-Konzentrationen zwischen 250 fM und 25 pM stimuliert; jede Spur stellt den Durchschnitt von vier [Ca²⁺]ᵢ-Messungen dar. Die Einzelspur (unteres Feld, blau) zeigt die Antwort der Spermien auf 25 pM Resact in Abwesenheit von extrazellulärem Ca²⁺ (11 mM EGTA in künstlichem Seewasser (*artificial seawater*, ASW)). **b:** Signale einer anderen Spermienprobe, gezeigt bei einer besseren Zeitauflösung für Resact-Konzentrationen zwischen 125 fM und 25 pM. **c:** Änderungen der [Ca²⁺]ᵢ in Spermien, stimuliert mit höheren Resact-Konzentrationen (25 pM bis 250 nM). **d:** Dieselben Aufzeichnungen wie in **c,** jedoch mit einer besseren Zeitauflösung dargestellt. **a, c,** und **d** stammen von derselben Spermienprobe. Die geringfügige Abnahme der ΔF₅₁₈ während der ersten ∼ 100 ms wurde durch den Mischvorgang verursacht. Die Abnahme wurde ebenfalls bei Kontrollmessungen (kein Resact) beobachtet und ist unabhängig von der Resact-Konzentration. **e:** Zeitverlauf der Ca²⁺-Antwort (2,5 pM Resact; schwarze Spur (mit IBMX); graue Spur (ohne IBMX); linke Ordinate) und des cAMP Anstiegs (Daten von Figur 1f; mit IBMX; rechte Ordinate).

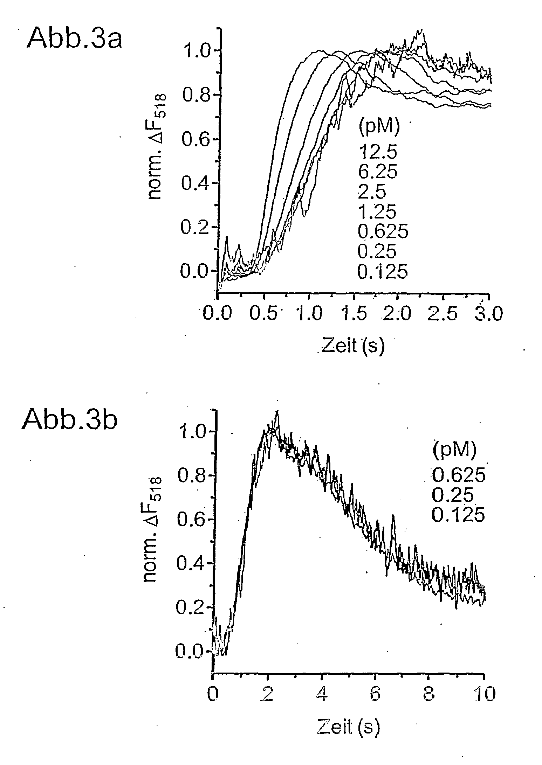

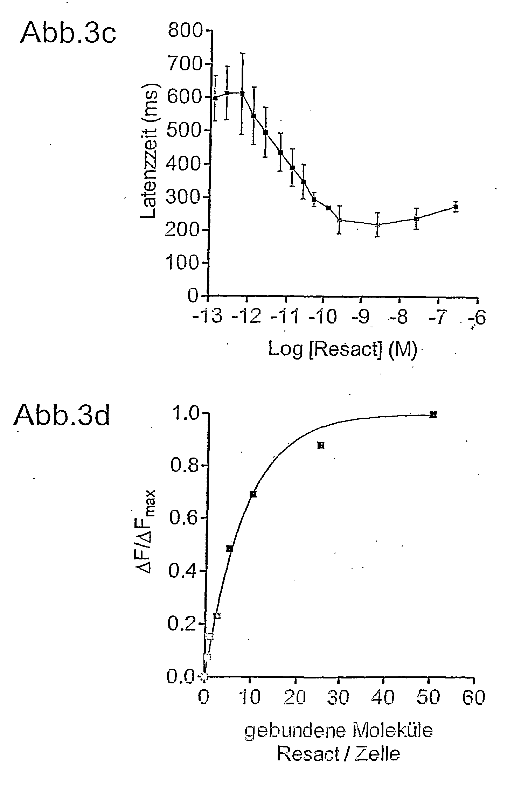

**Figur 3****:** Ca²⁺-Signale von Spermien auf einzelne Resact-Moleküle. **a:** Anfangsphase normalisierter Ca²⁺-Signale, hervorgerufen durch verschiedene Resact-Konzentrationen. **b:** Übereinander gelegte, normalisierte Ca²⁺-Signale, hervorgerufen durch Resact-Konzentrationen ≤ 625 fM. Die Ca²⁺-Signale in **a** und **b** wurden hinsichtlich des Mischartefakts korrigiert, indem die Kontrollsignale (ohne Resact) abgezogen wurden. **c:** Abhängigkeit der Verzögerung des Ca²⁺-Signals von der Resact-Konzentration. Die Verzögerung war definiert durch den Schnittpunkt der Regressionsgeraden, die den Anstieg der [Ca²⁺]ᵢ beschreibt, mit der Zeitachse. **d:** Abhängigkeit des frühen Ca²⁺-Signals (normalisierte Amplitude ΔF/ΔFₘₐₓ) von der Anzahl der gebundenen Resact-Moleküle pro Zelle. Die durchgehende Linie wurde kalkuliert nach Δ*F*/Δ*F*ₘₐₓ = 1 -e^{-kx}, wobei x die durchschnittliche Anzahl der gebundenen Resact-Moleküle pro Zelle und *k* eine Konstante bezeichnet, die charakteristisch für eine Spermienzelle ist. Die Anzahl der unabhängigen Segmente des Flagellums, 1/*k*, betrug in diesem Experiment 8,4 .

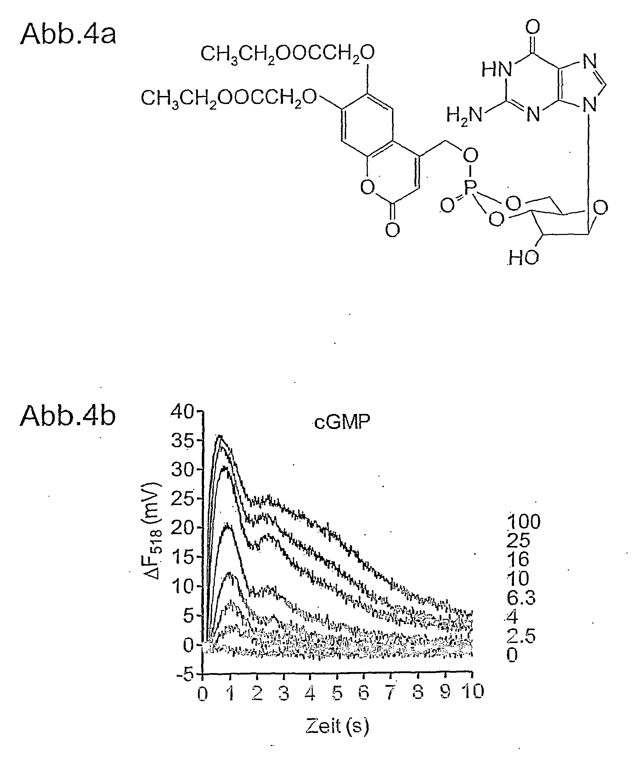

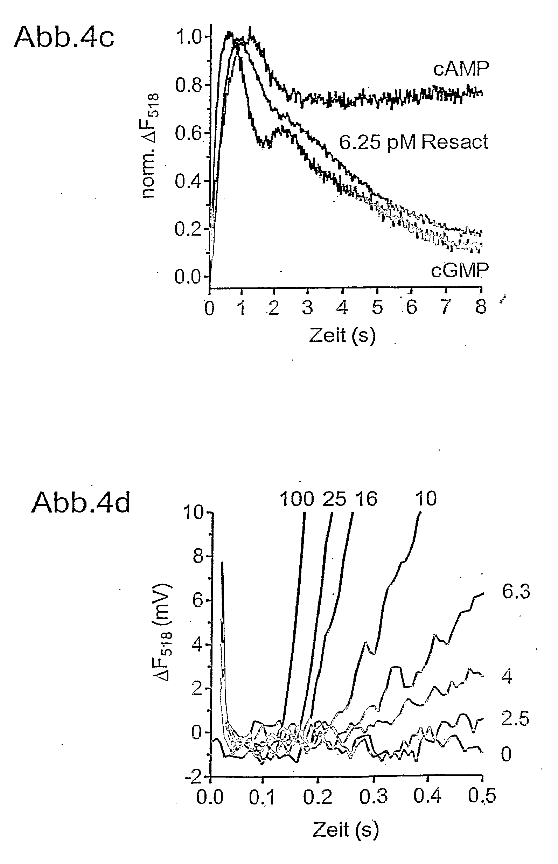

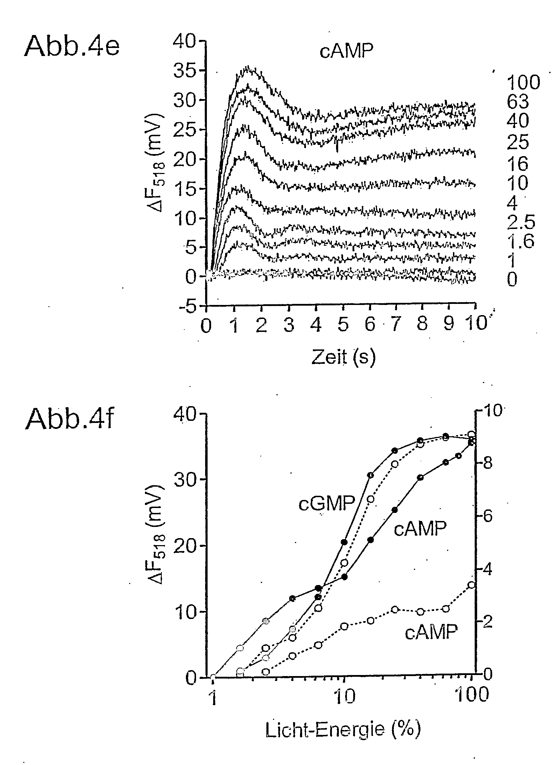

**Figur 4****:** Ca²⁺-Signale in Spermien, hervorgerufen durch zyklische Nukleotide. **a:** Chemische Struktur von caged-BECMCM-cGMP. **b:** Ca²⁺-Signale in Spermien, die mit caged-cGMP beladen waren. Die Ca²⁺-Signale wurden durch UV-Lichtblitze ausgelöst, durch die cGMP freigesetzt wurde. Die relative Lichtenergie der Blitze lag zwischen 2,5 % und 100 %. Die Energie eines Gesamtblitzes betrug 26,8 mJ cm⁻². **c:** Zeitverlauf normalisierter C²⁺-Signale, hervorgerufen durch Belichtung (16 % Blitz) von Spermien, die entweder mit caged-cGMP oder caged-cAMP beladen oder mit Resact (6,25 pM) stimuliert waren. Die Signale wurden auf der Zeitachse verschoben, um den unterschiedlichen Verzögerungszeiten Rechnung zu tragen. Die absoluten Amplituden der Signale betrugen 30,6 mV, 20,5 mV bzw. 18,7 mV. **d:** Ca²⁺-Signale aus b; die Signale sind bei einer besseren Zeitauflösung gezeigt, um zu verdeutlichen, dass die Verzögerungszeit von der relativen Menge des freigesetzten cGMPs abhängt. Das Signal am Anfang der Spuren zeigt den abfallenden Bereich des Blitzartefakts. **e:** Ca²⁺-Signale in Spermien, die mit caged-cAMP beladen waren. Das cAMP wurde durch Blitze verschiedener Energien (1 - 100 %) freigesetzt. **f:** Abhängigkeit der Amplituden der Ca²⁺-Signale von der relativen Blitzenergie für caged-cGMP und caged-cAMP (Daten aus b und e und einem zusätzlichen Experiment). Die gefüllten (linke Ordinate) und die leeren Kreise (rechte Ordinate) bezeichnen zwei Paare von Dosis-Wirkungs-Kurven aus elf Experimenten. Sie zeigen die Variabilität der cAMP-induzierten Ca²⁺-Signale.

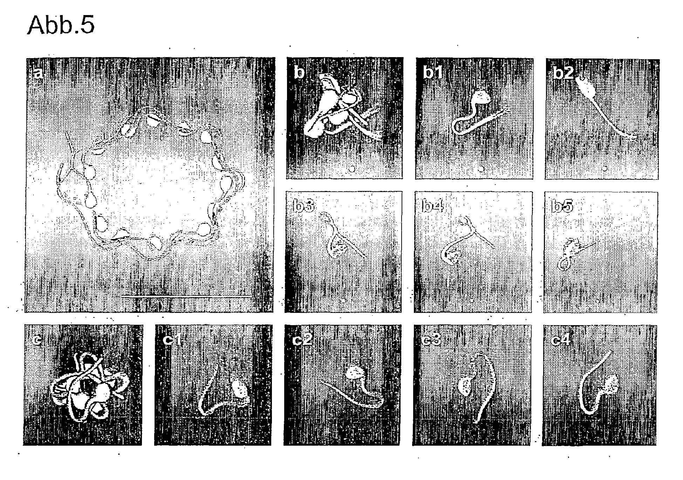

**Figur 5****:** Verhaltensmuster und Flagellenform der Spermien. **A**, Schwimmverhalten einer unstimulierten Zelle in ASW (80 ms Intervalle zwischen aufeinander folgenden Positionen). Der Durchmesser der Kreise betrug 56 ± 13 µm (Durchschnittswert ± Standardabweichung; n = 163). Die Schwimmgeschwindigkeit betrug 218 ± 30 µm s⁻¹. **b** and **c**, Taumeln und Schwimmen in engen Kreisen in Gegenwart von 10 pM Resact plus 0,5 mM IBMX; übereinandergelegte Bilder (**b**, **c**) und Einzelbilder (**b1-b5; c1-c4**) (40 ms Intervalle). Maßstab: 50 µm.

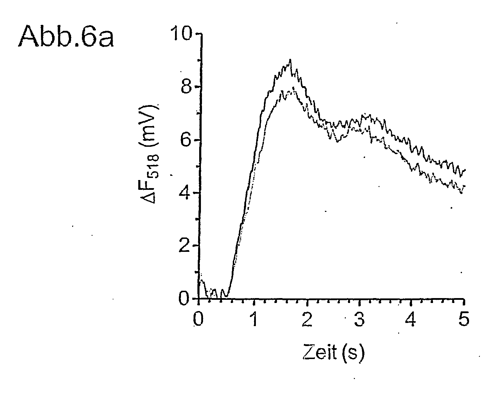

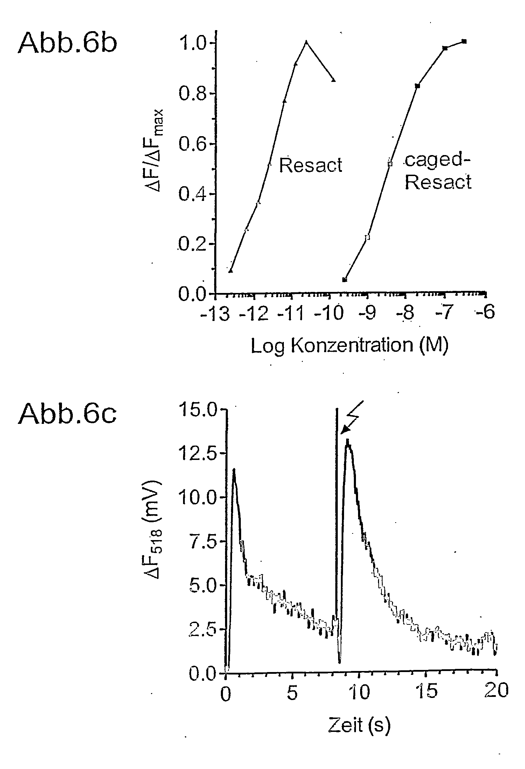

**Figur 6****:** Anstieg der [Ca²⁺]ᵢ in Spermien induziert durch Resact bzw. caged-Resact. **A**, Fluoreszenzsignale von Spermien, die mit Resact (1,25 pM, grau) bzw. caged-Resact (4 nM, schwarz) stimuliert waren. **B**, Normalisierte Dosis-Wirkungs-Kurven für Resact und caged-Resact; die K_{1/2} Werte betrugen 1,6 ± 0,6 pM (n=11) bzw. 4,9 ± 1,0 nM (n=5). C, Ca²⁺-Signale nach dem Mischen von Spermien mit caged-Resact (0,5 µm) und nach der Photolyse von caged-Resact mittels eines UV-Blitz.

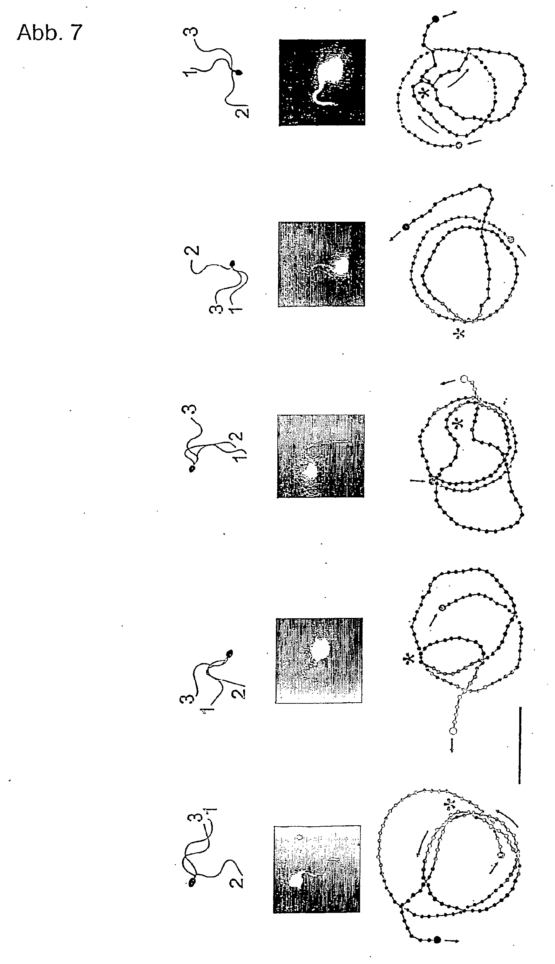

**Figur 7****:** Verhalten der Spermien nach Stimulation durch Resact, freigesetzt aus caged-Resact. *Untere Reihe*: Schwimmbahnen. Graue Bahnen: vor der UV-Belichtung, schwarze Bahnen: nach Einschalten der UV-Belichtung. Die Intervalle zwischen aufeinander folgenden Positionen betrugen 20 ms. Große Rasterpunkte markieren den Anfang und das Ende der Bahn. *Mittlere Reihe*: Einzelne Aufnahmen zeigen die Flagellenform während der ersten Wendereaktion (*). *Obere Reihe*: Übereinandergelegte Flagellenschlagmuster 80 ms vor (1), während (2) und 80 ms nach (3) der Wendereaktion. Die Konzentration von caged-Resact, der Anteil der reagierenden Zellen und die Reaktionszeit (Durchschnitt ± Standardabweichung) sind von links nach rechts: 100 pM, 8/45, 980 ± 415 ms; 1 nM, 48/62, 1048 ± 349 ms; 10 nM, 29/30, 610 ± 216 ms; 100 nM 63/71, 429 ± 63 ms; 1µM, 29/29, 337 ± 98 ms. Maßstab: 50 µm.

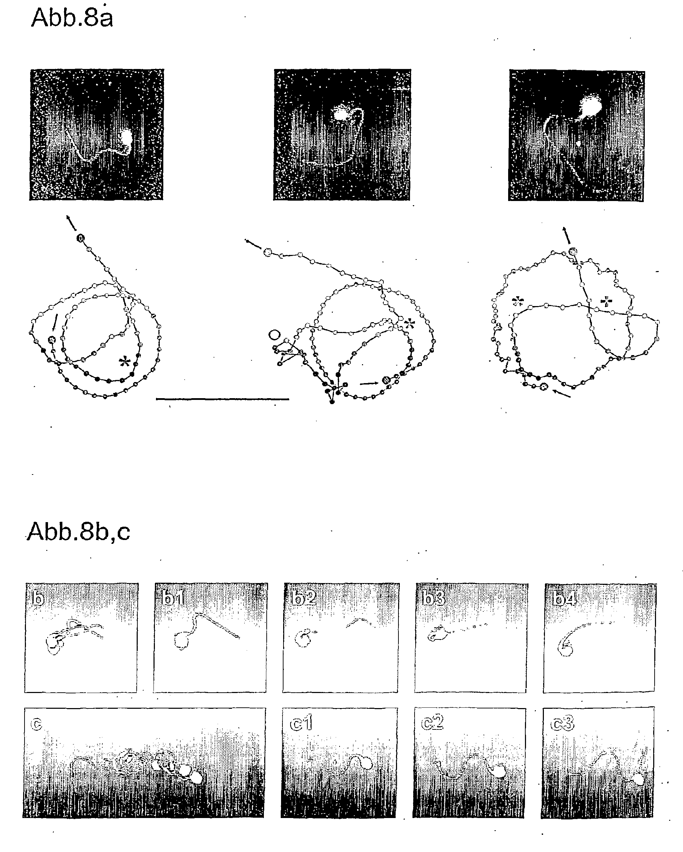

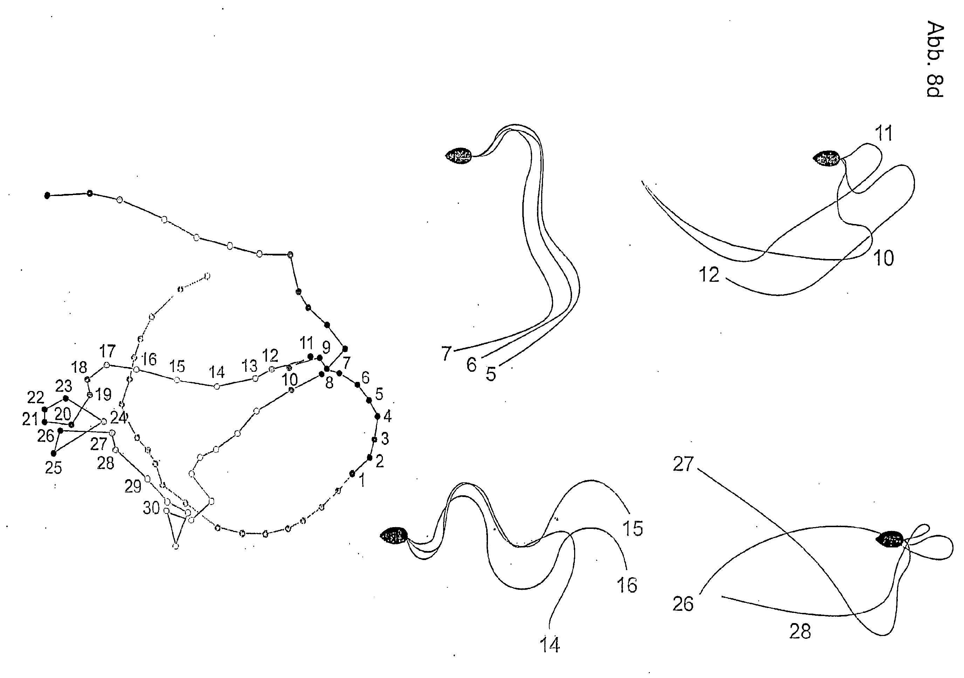

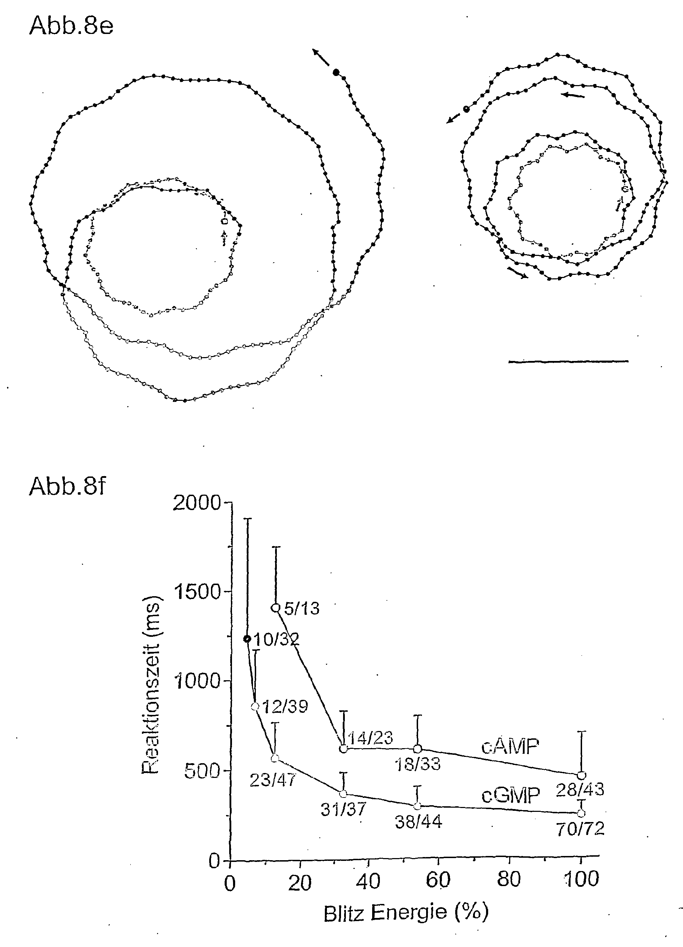

**Figur 8****:** Verhalten der Spermien nach Photolyse von caged-zyklischen Nukleotiden. **a,** Bahnen (20 ms Intervalle zwischen den Positionen) und Flagellenformen während der ersten Wendereaktion (*) ausgelöst durch cGMP. Graue Bahnen: vor der Photolyse, schwarze Bahnen: nach der Photolyse von caged-cGMP. Maßstab: 50 µm. **b,** Taumelbewegung aus der mittleren Bahn, die in **a** gezeigt ist - übereinandergelegt und in Einzelbildern (20 ms Intervall) in **b1-b4. c,** Eine Periode von geradlinigem Schwimmverhalten aus der rechten Bahn, die in a (+) gezeigt ist - übereinandergelegt und in Einzelbildern (20 - 40 ms Intervall) in **c1-c4. d**, Dieselbe Bahn wie in a (mittleres Bild) und übereinandergelegte Flagellenformen vor (5, 6, 7), während der ersten Wendereaktion (10, 11, 12), während des geradlinigen Schwimmens (14, 15, 16) und während des Taumelns (26, 27, 28). **E**, Schwimmbalmen nach Photolyse von caged-cGMP bei einer extrazellulären [Ca²⁺] < 10⁻⁵ M. Der Balken entspricht 50 µm. **f,** Reaktionszeit (Mittelwert ± Standardabweichung) für die cGMP- und cAMP-induzierten Wendereaktionen oder Taumelbewegungen in Abhängigkeit von der relativen Stärke des UV-Blitzes. Die Zahlen geben den Anteil der reagierenden Spermien an der Gesamtzellzahl an.

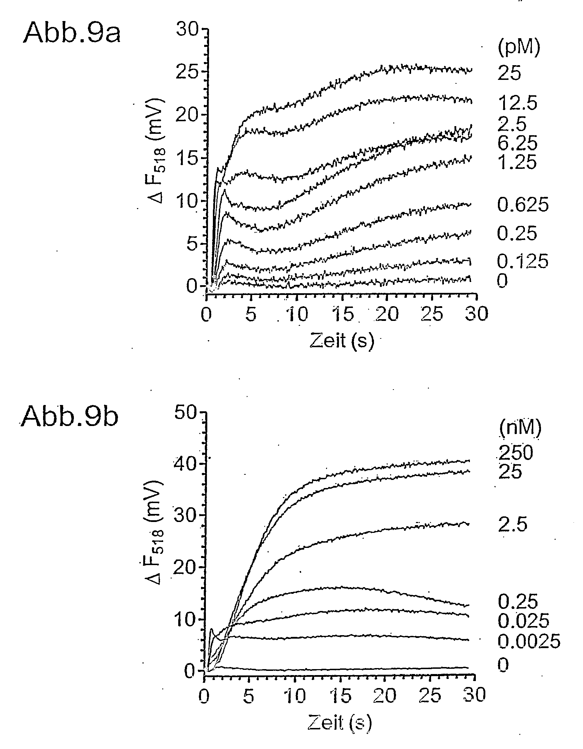

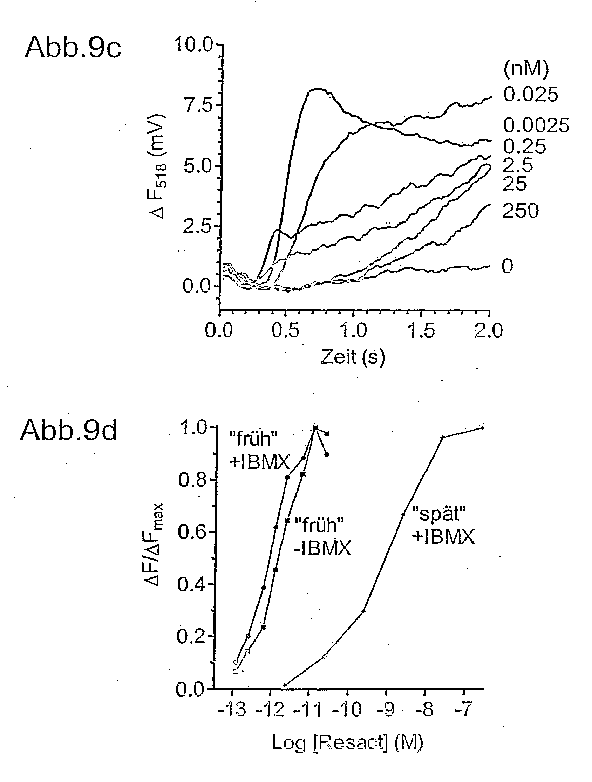

**Figur 9****:** Änderung der [Ca²⁺]ᵢ in Spermien, die in Gegenwart von 0,5 mM IBMX stimuliert wurden. **a,** 125 fM - 25 pM Resact und **b**, 2,5 pM - 250 nM Resact **c,** Spuren aus **b** gezeigt bei einer besseren Zeitauflösung. **d**, Normalisierte Dosis-Wirkungs-Kurve der frühen Ca²⁺ Antwort (Maximalwert) mit und ohne IBMX, und der späten Ca²⁺ Antwort in Gegenwart von IBMX.

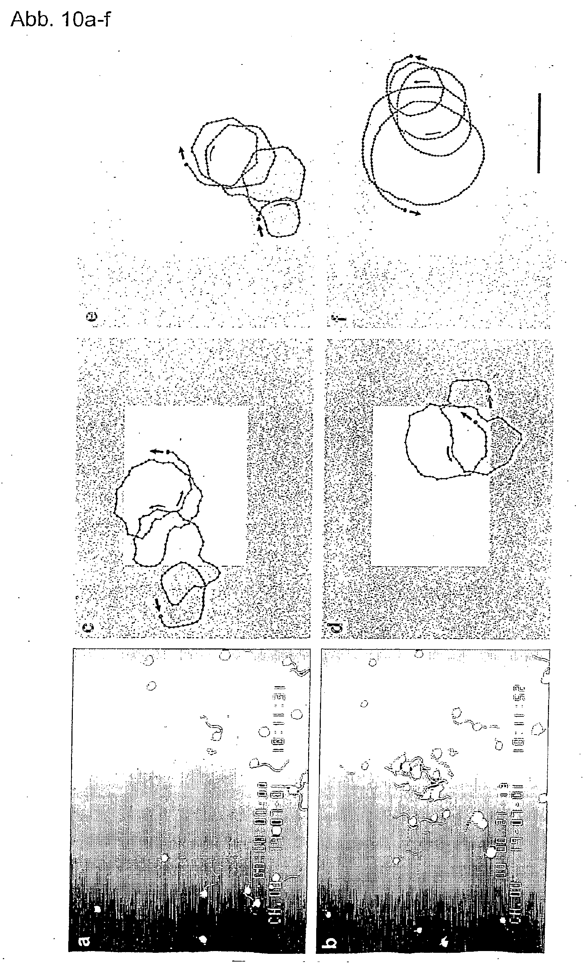

**Figur 10****:** Ansammlung von Spermien in einem rechteckigen Ausschnitt des Flüssigkeitsbehälters, in dem caged-Resact (10 µM) photolysiert wurde. **a,** Verteilung der Spermien vor der Photolyse. **b,** 21 s nach dem ersten und 10 s nach dem letzten von 10 UV-Blitzen. **c - f,** Schwimmbahnen einzelner Spermien (20 ms Intervalle zwischen aufeinander folgenden Positionen). Maßstab, 100 µm.

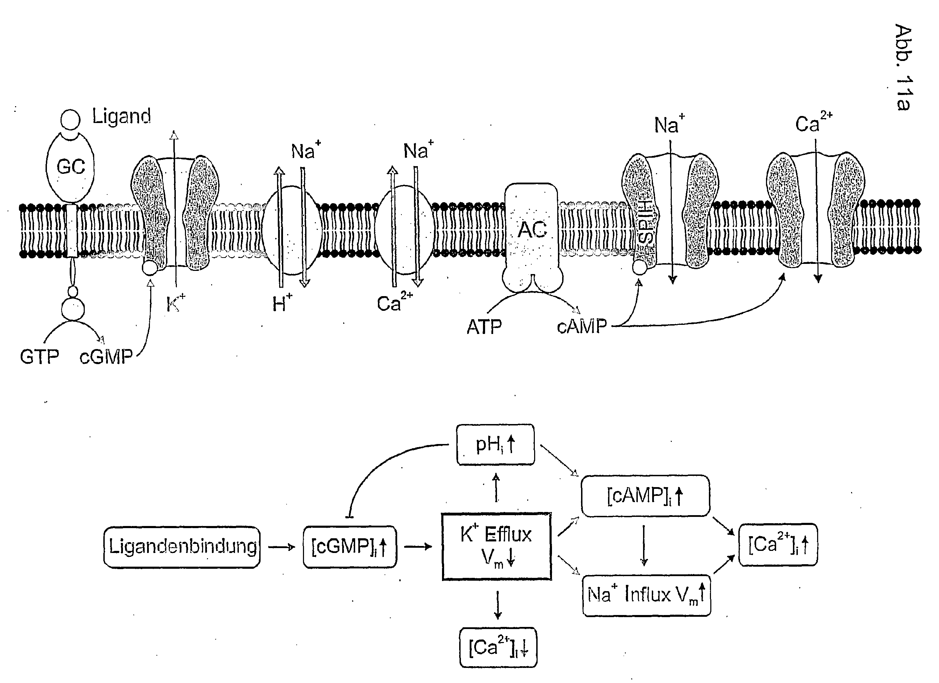

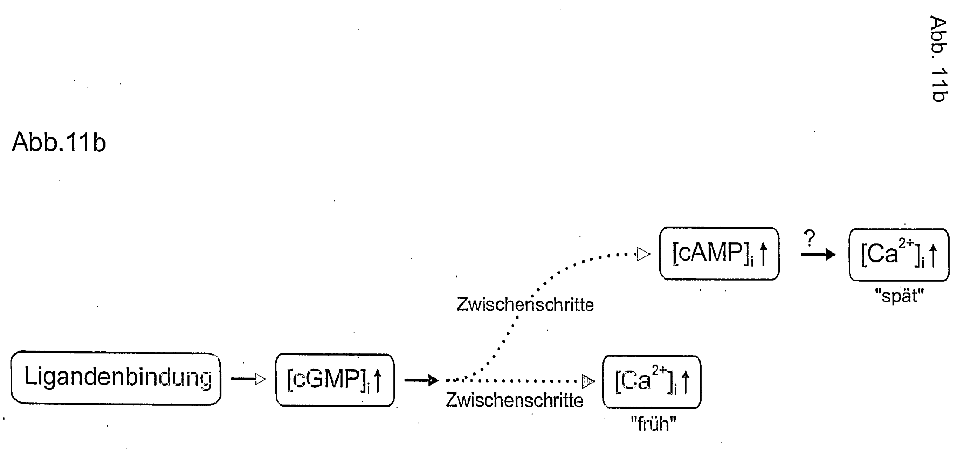

**Figur 11****:** Modelle des chemotaktischen Signalwegs **a**, Altes Modell. Durch die Bindung eines Eizellpeptids an eine Rezeptor-Guanylatzyklase auf der Oberfläche des Spermiums kommt es zu einem Anstieg der intrazellulären cGMP-Konzentration. Die Spermienzelle hyperpolarisiert, da cGMP-gesteuerte K⁺-Kanäle öffnen. Auf die Hyperpolarisation hin transportieren zwei Ionen-Transporter, ein Na⁺/H⁺-und ein Na⁺/Ca²⁺-Austauscher, Protonen bzw. Ca²⁺ -Ionen aus der Zelle hinaus; folglich steigt der intrazelluläre pH-Wert (pHᵢ) und die [Ca²⁺]ᵢ nimmt ab. In permeabilisierten Spermien korreliert die Asymmetrie des Flagellenschlags mit einer erhöhten [Ca²⁺]ᵢ; ein geradliniges Schwimmen erfolgt, wenn [Ca²⁺]ᵢ kleiner ist als die Ruhekonzentration von ungefähr 10⁻⁷ M. Die vermutete Abnahme der [Ca²⁺]ᵢ könnte dafür verantwortlich sein, daß in einem Lockstoffgradienten Spermien geradeaus auf die Lockstoffquelle zu schwimmen.. Wendereaktionen sollen erst sehr viel später auftreten, entweder dadurch, daß ein angestiegener pHᵢ oder eine Hyperpolarisation eine Adenylatzyklase aktiviert, wodurch die [cAMP] ansteigt. Der [cAMP]-Anstieg kann HCN-Kanäle (SPIH) aktivieren. Schließlich öffnet cAMP durch einen noch unbekannten Mechanismus Ca²⁺-permeable Kanäle: Ca²⁺-Ionen strömen in die Zelle ein und eine Wendereaktion wird ausgelöst. **b,** Neues Modell. cGMP öffnet, vielleicht indirekt, Ca²⁺-selektive Kanäle: der Anstieg der [Ca²⁺]ᵢ löst Wendereaktionen als erste Verhaltensantwort aus. Die [cAMP] nimmt erst während oder nach dem frühen Ca²⁺-Einstrom zu. Durch cAMP kann es zu einer länger anhaltenden Ca²⁺ Zunahme kommen, die die Empfindlichkeit des Spermiums für weitere Reize regulieren kann.

Die Erfinder haben ein Verfahren entwickelt, um die zellulären Reaktionen zu registrieren, die durch externe chemische Substanzen in Spermien ausgelöst werden. Die Substanzen können körpereigene und körperfremde Stoffe sein, die eine physiologische Funktion vermitteln, aber auch pharmakologische Wirkstoffe, die einen therapeutischen Nutzen haben könnten. Im folgenden werden die verschiedenen Variationen des Verfahrens beschrieben, mit dem man solche Substanzen identifizieren, nachweisen und charakterisieren kann.

Die zelluläre Stimulierung und Reaktion von Spermien mit externen Substanzen laufen sehr schnell ab. Bisherige Verfahren waren darauf beschränkt, Spermiensuspensionen und die Lösung, die den Wirkstoff enthält, relativ langsam, d.h. im Sekundenbereich, zu mischen. Die Erfinder haben mit Hilfe schneller Mischtechniken ("*Quenched-Flow*" und "*Stopped Flow*") Spermien und Wirkstoff schnell, d.h. in wenigen Millisekunden, gemischt und unterschiedliche zelluläre Reaktionen von Botenstoffen, z.B. Änderung der intrazellulären cGMP-Konzentration, Änderung der intrazellulären cAMP-Konzentration, Änderung der intrazellulären Ca²⁺-Konzentration und Änderung des pH-Wertes zeitaufgelöst im Sub-Sekundenbereich registriert..

Die vorliegende Erfindung betrifft daher ein Verfahren zum Nachweis von Änderungen von zellulären Botenstoffen in Spermien in einer Stopped-Flow-Messvorrichtung, enthaltend die Verfahrensschritte: wie in Anspruch 1 definiert.

Zelluläre Botenstoffe im Sinne der vorliegenden Erfindung sind cAMP, cGMP, Ca²⁺-Gehalt, pH-Wert, IP3, Membranpotential und zyklische ADP-Ribose, wobei diese Aufzählung nicht als abschließend angesehen werden kann.

Das Verfahren wird dabei in Messvorrichtungen durchgeführt, die nach dem *Stopped-Flow*- oder *Quenched-Flow*-Prinzip arbeiten und eine optische, vorzugsweise Fluoreszenz-optische Messvorrichtung aufweisen, mit dem der erfindungsgemäße Nachweis durchgeführt wird. Die Messvorrichtung kann zusätzlich eine Vorrichtung aufweisen, die in der Messkammer caged-Substanzen photolytisch freisetzen kann. Vorzugsweise wird das erfindungsgemäße Verfahren mit der *Stopped-Flow*-Vorrichtung der Firma BioLogic (SFM-4, BioLogic, Claix, Frankreich) durchgeführt.

Das *Stopped-Flow*-Verfahren ermöglicht es, den Zeitverlauf biochemischer Prozesse im Millisekunden- bis Sekundenmaßstab zu untersuchen (Johnson, 1986; Tareilus *et al*., 1995). Dieses Verfahren beruht darauf, Flüssigkeiten innerhalb kürzester Zeit zu mischen und die ablaufende Reaktion zu beobachten. Das Mischen der Flüssigkeiten bzw. der Reaktanden innerhalb kürzester Zeit, z.B. innerhalb von Millisekunden, vorzugsweise von weniger als 200 ms, vorzugsweise von weniger als 80 ms, vorzugsweise von weniger als 50 ms, besonders bevorzugt von weniger als 20 ms, besonders bevorzugt von weniger als 10 ms, insbesondere bevorzugt von weniger als 5 ms, ganz besonders bevorzugt innerhalb von weniger als 2 ms ist für das erfindungsgemäße Verfahren wichtig. Die Reaktionen können prinzipiell auf zweierlei Weisen verfolgt werden:
1) Das aus der Mischkammer fließende Reaktionsgemisch wird durch eine Beobachtungskammer geleitet und im kontinuierlichen Durchfluss beobachtet (*Continous-Flow*-Modus; Gutfreund, 1969). Wird die Reaktion zu einem bestimmten Zeitpunkt chemisch oder thermisch unterbrochen und das Reaktionsgemisch anschließend analysiert, so spricht man von dem *Quenched-Flow*-Modus (Tareilus *et al*. , 1995).
2) Der Flüssigkeitsstrom wird in der Beobachtungskammer (z.B. einer optischen Küvette) angehalten, um den Verlauf der Reaktion zu beobachten. Die Beobachtung wird z.B. mit Hilfe optischer, kaloriemetrischer oder elektrischer Meßverfahren durchgeführt. Diese Methode wird als *Stopped-Flow*-Modus bezeichnet (Gibson, 1969).

Ab welchem Zeitpunkt eine Reaktion beobachtet werden kann, nachdem die Reaktanden gemischt wurden, hängt sowohl von der Flussgeschwindigkeit (F) der Reaktanden als auch von dem durchfließenden Volumen (V) in der Messvorrichtung ab. Für die Dauer (T) der Reaktion bis zum Beobachtungs- bzw. Quenchzeitpunkt ergibt sich: T = V/F. Der Wert für (T) ist für das erfinderische Verfahren ein beliebiger Wert zwischen 5 und 1000 ms, vorzugsweise ein Wert zwischen 5 und 200 ms, besonders bevorzugt zwischen 10 und 100 ms, ferner besonders bevorzugt zwischen 20 und 50 ms, insbesondere bevorzugt zwischen 20 und 30 ms, ganz besonders bevorzugt bei 25 ms. Die Werte für (F) und (V) sind variabel und werden so gewählt, dass (T) einen Wert ergibt, der im genannten Bereich zwischen 5 und 1000 ms liegt. (T) kann jedoch auch die Dauer der Reaktion sein, die zeitlich nicht bestimmt sein muss z.B. bis zu 2 min betragen kann. Durch eine Veränderung der Flussgeschwindigkeit oder des Volumens der Reaktionskammern kann eine Reaktion zu verschiedenen Zeitpunkten beobachtet werden.

Die vorliegende Veröffentlichung betrifft ferner ein Verfahren zum Nachweis der Motilität oder Änderung des Schwimmverhaltens in Spermien in einem Flüssigkeitsbehältnis mittels einer mikroskopischen Vorrichtung und einer angeschlossenen Vorrichtung zur Bildaufzeichnung und -verarbeitung, enthaltend die folgenden Verfahrensschritte:
a) Aufzeichnen des Schwimmverhaltens von Spennien in dem Flüssigkeitsbehältnis vor Zugabe von Wirkstoff,
b) Mischen der Spermien und Wirkstoff bei gleichzeitigem Aufzeichnen des Schwimmverhaltens,
c) Aufzeichnen des Schwimmverhaltens der Spermien in dem Flüssigkeitsbehältnis nach Zugabe des Wirkstoffs und Auswerten der Änderung des Schwimmverhaltens.

Das Verfahren wird vorzugsweise bei einer Temperatur durchgeführt, die für die Spezies, von der die Spermien stammen, eine normale Umgebungstemperatur ist.

Die in den Verfahren verwendeten Spermien stammen von wirbellosen Tieren, insbesondere Insekten, die z.B. ökologische oder gesundheitliche Schäden bei Pflanzen, Tieren bzw. Menschen verursachen können, von Wirbeltieren, vorzugsweise von Säugetieren, Fisch oder anderen Meerestieren, besonders bevorzugt Spermien von Nutztieren, z.B. Schwein, Rind, Lamm, Pferd, Geflügel, Ratte, Kaninchen oder Maus, und insbesondere bevorzugt menschliche Spermien. Für eine einzelne Messung der zellulären Reaktionen werden etwa 10⁵ bis 10⁹ Spermien eingesetzt, vorzugsweise etwa 10⁶ bis 10⁸, besonders bevorzugt etwa 10⁷ Spermien. Es können jedoch auch weniger oder mehr Spermien verwendet werden.

Die Empfindlichkeit des Verfahrens ist so hoch, dass bei dieser Spermienzahl die Wirksamkeit einzelner Wirkstoffmoleküle pro Spermium noch nachgewiesen werden kann. Für die große Empfindlichkeit des Messverfahrens ist es wichtig, dass die Spermien nicht in einer Lösung mit unphysiologischem Ca²⁺-Gehalt oder pH-Wert vorliegen, wobei der physiologische Ca2⁺-Gehalt und pH-Wert von Spezies zu Spezies variieren kann. Ferner sollten die Spermien nicht für mehrere Stunden oder gar über Nacht inkubiert werden. Vorzugsweise sollten die Spermien nicht länger als etwa 2 Sunden insbesondere bevorzugt nicht länger als etwa 1 Stunde inkubiert werden. Weiterhin ist wichtig, dass die Spermiensuspension nicht abzentrifugiert und anschließend resuspendiert werden, sondern dass der Inkubationsansatz lediglich verdünnt wird. Dadurch wird eine Schädigung der Spermien verhindert.

Der verwendete Wirkstoff kann ein natürlicherweise vorkommender Lockstoff sein. So kann bei Untersuchung der Spermien des Seeigels *Arbacia punctulata* das Peptid *Resact* als Lockstoff verwendet werden. Bei Verwendung von Spermien anderer Organismen im Verfahren, werden bevorzugt die für diese Spennien natürlichen Lockstoffe verwendet. Ein Wirkstoff kann insbesondere auch jede Substanz, natürlich oder synthetisch, eine Aminosäurekette, z.B. ein Oligo- oder ein Polypeptid oder eine andere organische oder anorganische Substanz sein. Ein Wirkstoff kann des weiteren auch eine organische oder anorganische Stickstoffverbindung sein, aus der Stickoxid (NO) freigesetzt werden kann oder aber Stickoxid selbst. Ebenso kann ein Wirkstoff eine organische oder anorganische Kohlenstoffverbindung sein, aus der Kohlenmonoxid (CO) freigesetzt werden kann oder aber Kohlenmonoxid selbst. Bevorzugte Wirkstoffe sind ebenfalls cAMP und cGMP, sowie Derivate davon.

Der verwendete Wirkstoff muss jedoch nicht bekannt sein. Ein Wirkstoff ist auch eine Körperflüssigkeit, z.B. eine intrauterine oder intratubare, ein Zellextrakt oder einzelne Zellen, z.B. aus dem weiblichen Genitaltrakt, die auf das Vorhandensein von Lockstoffen oder Wirkstoffen mit agonistischer oder antagonistischer Wirkung durchmustert werden sollen.

Ferner können die Wirkstoffe in einem "künstlichen Käfig" eingeschlossen sein. Im Folgendem werden die so eingeschlossenen Verbindungen *caged* genannt. *Caged*-Wirkstoffe können insbesondere *caged*-cAMP, caged-cGMP, *caged*-Ca²⁺ und *caged*-Protonendonatoren und *caged-*Protonenakzeptoren sein. *Caged*-Wirkstoffe können aber auch *caged*-Lockstoffe sein, z.B. *caged*-Resact. Die *caged*-Wirkstoffe werden im erfindungsgemäßen Verfahren nach Zugabe und Mischen mit den Spermien durch Photolyse freigesetzt, woraufhin sie ihre Wirkung entfalten können.

Der "künstliche Käfig" ist eine chemische Verbindung, die mit dem Wirkstoff kovalent verknüpft ist. Vorzugsweise wird als *caged*-cAMP und *caged*-cGMP eine mit [6,7-bis(ethoxycarbonylmethoxy)coumarin-4-yl]methyl derivatisierte Form eingesetzt, die im Folgenden BECMCM-caged-cAMP und BECMCM-caged-cGMP genannt wird und die den jeweiligen Wirkstoff nach Belichtung mit UV-Licht schnell freigibt. Diese caged-Verbindungen durchdringen schnell die Zellmembranen, ergeben eine relativ große Quantenausbeute (ca. 0,1) und Absorbtivität, und sie sind besonders resistent gegenüber Solvolyse und setzen cAMP oder cGMP innerhalb von etwa 10 ns nach der Belichtung frei. Vorzugsweise wird ferner als *caged-*Lockstoff, z.B. *caged*-Resact eine mit [S-(4,5-dimethoxy-2-nitrobenzyl)Cys⁸] derivatisierte Form verwendet. Die vorliegende Publikation betriftt somit ferner cAMP und cGMP, kovalent verbunden mit [6,7-bis(ethoxycarbonylmethoxy)coumarin-4-yl]methyl und Resact, kovalent verbunden mit [S-(4,5-dimethoxy-2-nitrobenzyl)Cys⁸].

Der Nachweis der Änderung der Botenstoffe in Spermien, z.B. der cGMP-, cAMP-, IP3-, Ca²⁺-Konzentration, des Membranpotentials, der zyklischen ADP-Ribose oder des intrazellulären pH-Wertes wird im erfindungsgemäßen *Stopped-Flow*- oder *Quenched-Flow*-Verfahren insbesondere mit fluoreszierenden oder lumineszierenden Indikatoren durchgeführt. Mittlerweile ist ein Vielzahl solcher Indikatoren bekannt (Haugland RP, (2002) Handbook of fluorescent probes and research chemicals. Molecular Probes Inc.). Vorzugsweise wird die Änderung des pH-Wertes mit den Indikatoren SNARF, SNAFL oder deren Fluorescein-Derivaten, besonders bevorzugt mit BCECF (alle Molecular Probes; Eugene, USA) durchgeführt. Vorzugsweise wird die Änderung der Ca²⁺-Konzentration mit den Indikatoren Fluo-3, Fluo-5, Calcium Green, Fura-2 und besonders bevorzugt mit Fluo-4 (alle Molecular Probes; Eugene, USA) oder deren Fluorescein-Derivaten, sowie dem Calcium-Kit oder Calcium-Plus-Kit (Molecular Devices) durchgeführt.

Da solche Indikatoren normalerweise nicht wasserlöslich sind und deshalb die hydrophobe Lipidmembran von Zellen nicht passieren können, werden die Indikatoren in Form einer Esterverbindung, vorzugsweise als Acetoxymethylester (AM)-Verbindungen appliziert. Diese Verbindungen sind hydrophob und werden von Zellen aufgenommen. Im Zellinnern wird die Esterverbindung durch endogene Esterasen gespalten und der Indikator liegt in seiner wasserlöslichen Form vor. In dieser Form verbleibt er im Zellinnem und reichert sich dort an.

Der Nachweis der Änderung der Botenstoffe in Spermien, z.B. der intrazellulären cAMP- bzw. cGMP-Konzentration kann mittels eines radioaktiven, Lumineszenz- oder Fluoreszenz-optischen Nachweisverfahrens, z.B. Radioimmunoassays (RIA), *FlashPlate-, AlphaScreen- bzw. DELFIA-Assay System* (alle Perkin Elmer; Wellesley, MA, USA) und *Biotrak SPA-* oder *Biotrak EIA-Assay Kits* (beide Amersham Biosciences, UK) durchgeführt werden. Der Nachweis der Änderung der Botenstoffe in Spermien, z.B. der cGMP-, cAMP-, IP3-, Ca²⁺-Konzentration, des Membranpotentials, der zyklischen ADP-Ribose und des pH-Wertes in Spermien kann ebenfalls mittels eines Verfahrens zur Bestimmung der Änderung des Schwimmverhaltens durchgeführt werden.

Bevorzugt sind Verfahren, in dem Spermien mit einem Lockstoff, mit cGMP oder mit cAMP inkubiert werden und anschließend die Änderung der intrazellulären IP3-, Ca²⁺-Konzentration, des Membranpotentials, der zyklischen ADP-Ribose oder des pH-Wertes erfindungsgemäß nachgewiesen werden. Der Nachweis erfolgt vorzugsweise mittels des *Stopped-Flow*-Verfahrens mit dem Nachweisreagenz Fluo-4 oder mittels eines Verfahrens zur Bestimmung der Änderung der Beweglichkeit oder des Schwimmverhaltens. Die verwendeten Lockstoffe, das cGMP oder das cAMP sind vorzugsweise *caged*.

Aufgrund der Empfindlichkeit der erfindungsgemäßen Verfahren lassen sich einzelne Wirkstoffmoleküle und selbst geringste Wirkstoffgradienten nachweisen. Daher können mit den Verfahren nicht nur Wirkstoffbibliotheken durchmustert werden sondern es lassen sich auch endogene Wirkstoffe, z.B. Lockstoffe nachweisen und isolieren, die im weiblichen Genitaltrakt, z.B. in der Körperflüssigkeit, z.B. in der Follikelflüssigkeit, nur in minimalen Konzentrationen vorliegen und mit bisherigen Verfahren nicht nachgewiesen werden konnten.

Die Verfahren können verwendet werden, um endogene physiologische Faktoren, z.B. Lockstoffe des weiblichen Genitaltrakts, zu identifizieren und isolieren. Ferner kann das Verfahren verwendet werden, um synthetische oder natürliche Agonisten oder Antagonisten der Wirkstoffe zu identifizieren. Vorzugsweise werden dafür Wirkstoffbibliotheken durchmustert, die sowohl synthetische als auch natürliche Substanzen enthalten können. Insbesondere können nach Sequenzierung des menschlichen Genoms oder auch anderer Genome die translatierten Sequenzen auf ihre Wirkung als Lockstoff bzw. auf ihre agonistische oder antagonistische Wirkung untersucht werden. Vorzugsweise dienen die Verfahren dazu, einen oder mehrere Wirkstoffe im weiblichen Genitaltrakt zu identifizieren, die die Beweglichkeit, das Schwimmverhalten, insbesondere das zielgerichtete Schwimmverhalten von Spermien steuern und/oder modulieren und/oder den Befruchtungsvorgang beeinflussen. Solche Wirkstoffe selbst können zur Steigerung der Fertilisation oder zur Kontrazeption eingesetzt werden. Sie können aber auch als Leitsubstanzen für die Entwicklung von pharmakologischen Substanzen dienen, die die vorstehend beschriebenen Eigenschaften der Wirkstoffe aufweisen. Leitsubstanzen können beispielsweise Peptide, Oligopeptide, Polypetide oder andere organische Moleküle umfassen, sowie anorganische Moleküle wie beispielsweise Stickoxid (NO) oder Kohlenmonoxid (CO), bzw. Substanzen, aus denen NO bzw. CO freigesetzt werden können. Sie können mit Hilfe chemischer, biochemischer oder molekularbiologischer Methoden so verändert werden, dass sie eine verbesserte pharmakologische Wirkung aufweisen, insbesondere hinsichtlich ihrer Spezifität, Affinität, Verträglichkeit oder ihrer Applikationsmöglichkeiten (z.B. orale, intrauterine Applikation). Die Basis für solche Veränderungen kann ebenfalls das computerunterstützte "Modellieren" (*modelling*) neuer Wirkstoffe bilden.

Ein weiterer Aspekt der vorliegenden Erfindung betrifft die Verwendung der erfindungsgemäßen Verfahren zur Entwicklung und Charakterisierung von Diagnostika oder diagnostischen Test-Kits, z.B. diagnostischen "Schnelltests", mit denen die Vitalität und/oder Fertilität von Spermien und Eizellen bestimmt werden kann.

Des weiteren kann das Verfahren für die Entwicklung von Diagnostika oder diagnostischen Test-Kits, z.B. diagnostischen "Schnelltests" verwendet werden, mit denen Lockstoffe von Eizellen oder andere Faktoren im weiblichen Genitaltrakt, die die Fertilität fördern oder vermindern können, identifiziert, charakterisiert oder nachgewiesen werden.

Die folgenden Beispiele erläutern die Erfindung und sind nicht als einschränkend aufzufassen.

Seeigel-Spermien wurden entnommen, indem 0,5 - 1 ml 0,5 M KCl in das Coelom des Seeigels injiziert wurden. Alternativ wurde der Seeigel durch elektrische Reizung zum Absamen stimuliert. Hierzu wurde mittels zweier Elektroden eine Spannung von ca. 25 V an das Tier angelegt. Das austretende Sperma bezeichnet man als *dry sperm. Dry sperm* wurde mit künstlichem Seewasser (ASW, in mM: 423 NaCl, 9,27 CaCl₂, 9 KCl, 22,94 MgCl₂, 25,5 MgSO₄, 0,1 EDTA, 10 HEPES-NaOH, pH 7,8) verdünnt. Die Zellzahl wurde in einer Neubauer-Zählkammer bestimmt.

Die Beweglichkeit der Spermien von *Arbacia punctulata* (Seeigel) und *Asterias amurensis* (Seestern) wurde als ein Kriterium für ihre Vitalität genommen. 100 µl einer verdünnten Spermienstammsuspension wurden auf einen Objektträger pipettiert und die Spermien wurden im Phasenkontrast mikroskopiert. Ca. 90% der Spermien waren motil. Grundsätzlich wurde bei *A. punctulata* nur *dry sperm* verwendet, das weniger als 6 h alt war.

### Beispiel 1: Messung von Änderungen in der intrazellulären Ca²⁺-Konzentration

Resact-induzierte Änderungen in der intrazellulären Ca²⁺-Konzentration wurden mit der schnellen Mischvorrichtung im *Stopped-flow*-Modus unter Verwendung des Ca²⁺-Fluoreszenzindikators Fluo-4 gemessen. Das Kernstück der *Stopped-Flow*-Apparatur war das SFM-4-Modul, das vier Spritzen (S1, S2, S3 und S4) enthielt. Die Spritzen wurden jeweils über ein Ventil durch externe Eingänge mit den zu mischenden Flüssigkeiten gefüllt. Die Stempel der Spritzen wurden durch Schrittmotoren gesteuert. Die Schrittmotoren wurden durch einen Personal computer (PC) (Pentium II, 64MB RAM) über eine Steuerungseinheit (MPS-52, BioLogic) angesteuert. Als Steuerungssoftware diente die MPS-Software (V.1.21, BioLogic). Das Ausstoßvolumen der Spritzen betrug 4,54 µl pro Schritt. Die Flussgeschwindigkeiten lagen zwischen 0,05 und 8,00 ml/s bei den 20 ml Spritzen. Mit Hilfe der *Stopped-Flow-Apparatur* konnten vier verschiedene Lösungen gemischt werden. Die Lösungen wurden von den Spritzen S1 - S4 in die verschiedenen Mischkammern (M1 - M3) gepresst. Zwischen den Mischkammern wurden bei Bedarf zwei Reaktionskammern (I, II) mit unterschiedlichen Reaktionsvolumina installiert. Die Reaktionskammern waren in Edelstahl- oder spezielle Kunststoffblöcke gebohrt, die man in unterschiedlichen Kombinationen in das Röhrensystem einbauen konnte. Ohne die Reaktionskammern I und II betrug das Volumen der Röhren zwischen M1 und M2 17-µl bzw. zwischen M2 und M3 22 µl. Von der Mischkammer M3 floss das Reaktionsgemisch in die Küvette. Als Beobachtungskammer für den Zeitverlauf der Reaktion diente eine Quarzküvette (FC-15) mit 31 µl Reaktionsvolumen. Um Schwankungen der Flüssigkeitssäule in der Küvette und dadurch Schwankungen des Messsignals zu verhindern, wurde ein *hard-stop*-Ventil hinter der Küvette direkt nach der Injektion einer Flüssigkeit in die Messküvette geschlossen. Das Ventil wurde über die Steuerungssoftware der *Stopped-Flow*-Apparatur angesteuert. Die Zeitspanne, die zwischen dem Mischen der Reaktionspartner und dem Beobachtungsbeginn vergeht, wird als sogenannte "Tot-Zeit" (T) bezeichnet. Die Tot-Zeit hängt von zwei Faktoren ab: 1. Von der Flussgeschwindigkeit (F), mit der das Reaktionsgemisch von der Mischkammer in die Küvette fließt, und 2. von dem Volumen (V), durch welches das Reaktionsgemisch fließen muss. Um eine möglichst geringe Tot-Zeit zu erreichen, wurden die beiden Reaktionspartner in Mischkammer M3 gemischt. Aus Spritze 3 bzw. 4 wurde SAP (*sperm activating peptide*)-haltiges ASW bzw. die Spermien-Suspension in die Mischkammer injiziert und dort in weniger als 1 ms gemischt. Spritze S 1 wurde mit Puffer - in diesem Fall ASW - befüllt, der zum Spülen der Mischkammern und der Küvette diente. Für die Fluoreszenzmessungen wurden keine Reaktionskammern in die *Stopped-Flow*-Apparatur eingebaut.

Die Flussgeschwindigkeit, mit der das Reaktionsgemisch von der Mischkammer in die Küvette floss, wurde durch ein sogenanntes "Spritzenprogramm" bestimmt. Dieses Spritzenprogramm wurde mit dem MPS-Programm im PC entworfen. Ein Experiment wurde dabei in einzelne Phasen unterteilt. Im Programm wurde die Dauer der einzelnen Phasen festgelegt. Außerdem wurde festgelegt, aus welcher Spritze welches Volumen in die jeweilige Mischkammer ausgestoßen wird. Aus dem gesamten Ausstoßvolumen einer Phase und der Phasendauer ergab sich die Flussgeschwindigkeit. Indem man den Parameter *Synchro 1* auf *on* stellte, bestimmte man den Zeitpunkt, an dem das vom Photomultiplier gemessene Signal aufgezeichnet wurde. Die Dauer der Aufnahme sowie andere Parameter der Aufnahme wurden mit Hilfe der BioKine-Software bestimmt, welche unabhängig von der Programmierung der MPS-Software war.

**Tabelle 1**

| Phase | 1 | 2 | 3 | 4 | 5 | 6 |
|---|---|---|---|---|---|---|
| Phasendauer (ms) | 50 | 200 | 300 | 200 | 1300 | 10 |
| Spritze 1 (µl) (ASW) | | 500 | | | | |
| Spritze 2 (µl) (-) | | | | | | |
| Spritze 3 (µl) (ASW mit SAP) | | | | 100 | | |
| Spritze 4 (µl) (Spermiensuspension) | | | | 100 | | |
| Synchro 1 | On | Off | Off | Off | Off | Off |
| Synchro 2 | Off | Off | Off | Off | Off | On |

**Tab. 1:** Beispiel eines Spritzenprogramms für die *Stopped-Flow*-Messung. Phase 1 dauerte 50 ms und diente dazu, die Schrittmotoren der Spritzen zu synchronisieren und die Aufzeichnung des Messsignals mit Hilfe eines Photomultipliers zu starten. Phase 2 dauerte 200 ms. Während dieser Zeit wurde ein Volumen von 500 µl aus der Spritze S 1 in das Röhrensystem gepresst, um die Reste von Reaktanden der vorherigen Messung wegzuwaschen. Die Flussgeschwindigkeit betrug 2,5 ml/s. Die Flüssigkeit verblieb für 300 ms im Röhrensystem (Phase 3). In Phase 4 wurden jeweils 100 µl aus Spritze 3 und 4 mit einander gemischt. Da die Phasendauer 200 ms war, betrug die Flussgeschwindigkeit 1,0 ml/s. Die Tot-Zeit betrug 31 ms.

Um eine Veränderung der intrazellulären Ca²⁺-Konzentration mittels *caged*-Verbindungen zu bestimmen, wurde die Photolyse der *caged*-Verbindungen durch eine Blitzlampe, die über einen Lichtleiter an die Messküvette gekoppelt war, zu einem beliebigen Zeitpunkt ausgelöst. Dazu wurde in Phase 6 *Synchro 2* auf *on* gestellt, so dass der Lichtblitz mittels eines TTL-Pulses ausgelöst wurde. Phase 5 diente nur dazu, den Zeitpunkt des Blitzes zu bestimmen. Das lichtinduzierte Spannungssignal der Photomultiplier-Röhre wurde über die Photomultiplier-Detektions-Einheit an einen PC mit integriertem A/D-Wandler (DT 2801-A, Bio-Logic) weitergeleitet und dann mit der BioKine-Software (Version 3.23, Bio-Logic) aufgezeichnet und bearbeitet.

*Dry sperm* wurde im Verhältnis 1:6 in Beladungspuffer aufgenommen, der ASW, 10 µM Fluo-4AM und 0,5 % Pluronic F127 (Sigma) enthielt. Nach 45-120 min Inkubation mit Fluo-4AM bei 17°C wurde die Probe 1:20 mit ASW bei pH 7,5 oder pH 7,8 verdünnt. Es ergaben sich keine oder nur sehr geringe Unterschiede in den Ergebnissen, die bei pH 7,5 oder pH 7,8 erzielt wurden. Die Spermien wurden dann 5 min mit dem neuen Medium equilibriert. In der *Stopped-flow*-Vorrichtung wurde die Spermienlösung schnell mit ASW-Lösungen, die verschiedene Konzentrationen von Resact enthielten, im Verhältnis 1:1 gemischt. Die Spermiendichte in der Quartzküvette betrug 0,9 bis 4,4 x 10⁸ Zellen/ml. Die Zellen wurden innerhalb der nächsten ca. 15 min für die *Stopped-Flow*-Experimente verwendet. Für die große Empfindlichkeit des Messverfahrens ist es wichtig, dass die Spermien in ASW mit einem Ca²⁺-Gehalt ≥ 9 mM und/oder mit einem pH-Wert ≥7,5 und ebenfalls nicht für mehrere Stunden oder gar über Nacht inkubiert werden. Weiterhin ist wichtig, dass die Spermiensuspension nicht abzentrifugiert und die Spermien resuspendiert werden, sondern dass der Inkubationsansatz lediglich verdünnt wird. Dadurch wird eine Schädigung der Spermien verhindert.

Aufgrund der nachfolgenden Gründe wurde nicht versucht, ΔF₅₁₈ in Werte der freien intrazellulären Ca²⁺-Konzentration umzurechnen. Konfokale Bilder von einzelnen Spermien, die mit Fluo-4 beladen waren, ergaben ein heterogenes Fluoreszenzmuster. Die Fluoreszenz von unstimulierten Spermien war im Bereich intrazellulärer Ca²⁺-Speicher (Mitochondrien und Akrosomvesikel) verstärkt. Des weiteren sind Spermien hoch polarisierte Zellen und die Orte, an denen Ca²⁺ einströmt, sind wahrscheinlich nicht gleichmäßig verteilt. Änderungen in ΔF₅₁₈, die auf vielen Spermien in einer Spermienlösung basieren, ergeben räumliche Durchschnittswerte. Daher können aussagefähige Werte für die freie intrazelluläre Ca²⁺-Konzentration in Spermien nicht von Standard-Kalibrierungsverfahren abgeleitet werden.

Die *Stopped-Flow*-Apparatur wurde mit einem Messsystem erweitert, dass es erlaubte, Fluoreszenz in der Küvette zu messen. Als Quelle für das Anregungslicht der Ca²⁺- und pH-Indikatoren diente eine 150 Watt Xe/Hg-Lichtbogenlampe (Hamamatsu, Japan). Um die Wellenlängen zur Anregung der Ca²⁺- und pH-Indikatoren auszuwählen, wurden zwei FITCA-40 Interferenzfilter (2 mm; Schott) verwendet. Vor die Interferenzfilter wurden ein Wärmeschutzfilter (3 mm; KG1, Schott) und zwei 400 nm Langpassfilter (3 mm; GG400, Schott) eingebaut. Diese Filter schützen die Interferenzfilter vor einer Beschädigung durch Wärme bzw. vor der Solarisation durch UV-Licht. Die Fluoreszenz wurde mit einem Photomultiplier (HTV-R376, Hamamatsu, Japan) detektiert, der im rechten Winkel zum Strahlengang des Anregungslichts installiert wurde. Bei den Ca²⁺- und pH-Messungen wurden zwischen der Küvette und dem Photomultiplier zwei 495 nm Langpassfilter (3 mm; GG495, Schott) eingebaut, die verhindern sollten, dass an den Spermien gestreutes Anregungslicht auf den Photomultiplier gelangt. Des weiteren wurden zwei FITCE-45 Interferenzfilter (2 mm; Schott) verwendet. Diese verhinderten, dass Anregungslicht, das langwelliger als das Fluoreszenzlicht der Ca²⁺- oder pH-Indikatoren war, vom Photomultiplier detektiert wurde. Wenn nicht anders angegeben, wurde für die Messungen der Ca²⁺- und pH-Signale eine Spannung von 500 V an den Photomultiplier angelegt. Die Änderung im Photomultiplierauslaß war linear mit den Änderungen der Fluoreszenz.

### Resact aktiviert frühe und späte Ca²⁺-Signale

Spermien, die mit dem Ca²⁺-Indikator Fluo-4 beladen waren, antworteten mit einem transienten Anstieg der Fluoreszenz (Fig. 2, ΔF₅₁₈) nach Stimulierung mit Resact. In Anwesenheit von EGTA ([Ca²⁺] < 10⁻⁶ M) hingegen wurde keine Resact-stimulierte ΔF₅₁₈ festgestellt (Fig. 2a, unteres Feld). Daher zeigt ΔF₅₁₈ einen Anstieg der intrazellulären Ca²⁺-Konzentration, hervorgerufen durch einen Ca²⁺-Einstrom in die Zelle. Es fand sich kein Hinweis für eine frühe Resact-stimulierte Abnahme der intrazellulären Ca²⁺-Konzentration (zeitliche Auflösung von etwa 10 ms).

Die Form und der Verlauf der Ca²⁺-Signale waren abhängig von der Resact-Konzentration. Nach einer kurzen Verzögerungszeit stieg die intrazelluläre Ca²⁺-Konzentration auf einen Spitzenwert an und fiel dann auf einen niedrigeren Plateauwert ab, dessen Höhe mit der Resact-Konzentration korrelierte (Fig.2a, b). Die Verzögerungszeit und die Anstiegszeit wurden jeweils mit ansteigender Resact-Konzentration geringer (Fig. 2b). Die kleinste Verzögerungszeit betrug etwa 200 ms (Fig. 4c). Diese Ergebnisse lassen vermuten, dass ein Teil der Verzögerung dadurch zustande kommt, dass cGMP synthetisiert wird. Die Abklingphase des Ca²⁺-Signals war durch eine Schulter charakterisiert (Fig. 2b), die für Resact-Konzentrationen ≥ 125 pM sich zu einem zweiten, lang anhaltenden Ca²⁺-Anstieg entwickelte (Fig.2c). Die zwei kinetischen Komponenten des Fluoreszenzsignals wurden als "frühe" und "späte" Ca²⁺-Antwort bezeichnet.

Eine Resact-Konzentration von 125 fM rief durchweg die frühe Ca²⁺-Antwort hervor. Die Antwort war maximal zwischen 12,5 und 25 pM Resact und verringerte sich bei höheren Konzentrationen (Fig.2d). Das frühe Ca²⁺-Signal nahm bei höheren Resact-Konzentrationen schneller ab (außer bei 250 nM) (Fig. 2c, d). Diese Beobachtungen deuten an, dass die Ca²⁺-Aufnahme durch einen negativen Feedback-Mechanismus abgeschwächt wird, der mit höheren Resact-Konzentrationen schneller wird.

IBMX (0,5 mM) verhinderte fast vollständig, dass das frühe und das späte Ca²⁺-Signal wieder zurückgingen (Fig . 9a, b). Dies deutet darauf hin, dass die Erholung von der Stimulierung durch die Hydrolyse von zyklischen Nukleotiden hervorgerufen wird. IBMX enthüllte drei weitere Merkmale der Ca²⁺-Antwort. Erstens: Bei hohen Resact-Konzentrationen wurde die frühe Antwort durch IBMX stärker unterdrückt als ohne IBMX (Fig. 9c). Das deutet darauf hin, dass cGMP in eine negative Rückkopplung involviert ist, die die Ca²⁺-Antwort abschwächt. Zweitens: Die späte Ca²⁺-Antwort fiel bei hohen Resact-Konzentrationen viel größer aus als die frühe Ca²⁺-Antwort (vgl. Fig. 2c und Fig. 9c). Die unterschiedlichen Effekte von IBMX erlaubten es, die Empfindlichkeit der frühen und der späten Ca²⁺-Antwort für Resact zu bestimmen. Der K_{1/2}-Wert der späten Ca²⁺-Antwort war um den Faktor 500 größer als der der frühen Ca²⁺-Antwort (1000 pM ± 870 (n = 3) und 1,8 ± 1,3 pM (n = 3) (Fig. 9d). Die K_{1/2}-Werte der frühen Ca²⁺-Antwort in Gegenwart und Abwesenheit von IBMX waren ähnlich. Drittens: IBMX verzögerte den Anstieg der cAMP-Konzentration; die Anstiegszeit der frühen Ca²⁺-Antwort hingegen wurde durch IBMX verkürzt (Fig. 2e). Diese Beobachtung ist unvereinbar mit einer Rolle von cAMP in der frühen Ca²⁺-Antwort. Die Verstärkung der späten Ca²⁺-Antwort durch IBMX kann dafür verantwortlich sein, dass die Akrosom-Reaktion durch hohe Peptid-Konzentrationen und PDE-Inhibitoren ausgelöst werden kann.

### Spermien erkennen einzelne Resact-Moleküle

Um die Empfindlichkeit von Spermien für Resact zu bestimmen, wurde die Anzahl der gebundenen Resact-Moleküle ermittelt. Die Anzahl der gebundenen Resact-Moleküle wurde durch Mischen der Resact-Lösungen mit Spermien bestimmt (∼ 1 bis 5x10⁸Zellen/ml, entsprechend 2,3 bis 11,7 nM Resact-Rezeptoren; Oberflächendichte 14.000 pro Zelle). Nachdem gebundenes von freiem Resact durch Zentrifugation der Spermien getrennt war, wurde der Überstand in der *Stopped-flow-*Vorrichtung mit Fluo-4-beladenen Spermien gemischt. Um die Resact-Konzentration in dem Überstand zu bestimmen, wurde die damit erhaltene Ca²⁺-Antwort mit Ca²⁺-Antworten verglichen, die mit definierten Resact-Konzentrationen erzielt wurden. Für Resact-Konzentrationen von bis zu ungefähr 250 pM wurden alle Resact-Moleküle gebunden, was mit der extrem hohen Dichte und Affinität der Resact-Rezeptoren in den Spermien übereinstimmt. Daher ist die Gesamtzahl der zugefügten Resact-Moleküle gleich der Anzahl der gebundenen Moleküle. Bei einer Lösung von 500 fM Resact (∼ 3x10⁸ Moleküle/ml) kommt daher bei einer Spermiendichte von etwa 3x10⁸/ml durchschnittlich ein gebundenes Resact-Molekül auf ein Spermium.

Falls das Fluoreszenzsignal für Resact-Konzentrationen von ≤ 1pM den Gesamtdurchschnitt der einzelnen Molekül-Antworten repräsentiert, sollte die Verzögerungszeit und Form von normalisierten Ca²⁺-Signalen identisch und unabhängig von der Resact-Konzentration sein. Tatsächlich stimmen die Zeitverläufe für normalisierte Antworten für Resact-Konzentrationen bis etwa 0,5 pM überein, also wenn weniger als 1 Resact-Molekül pro Spermium gebunden ist. Erst bei höheren Resact-Konzentrationen ändert sich der Zeitverlauf (Fig. 3a-c). Diese Ergebnisse zeigen, dass Spermien einzelne Moleküle erkennen.

Die frühe Ca²⁺-Antwort war maximal bei 12,5 - 25 pM Resact (etwa 50 - 100 Resact-Moleküle gebunden pro Zelle, was 0,35 % - 0,7 % belegter Rezeptoren entspricht). Daher kann eine Sättigung der Rezeptoren mit dem Lockstoff nicht für die Sättigung der Ca²⁺-Antwort verantwortlich sein. Die Beziehung zwischen der Größe der Ca²⁺-Antwort und der Resact-Konzentration wird gut durch eine exponentielle Sättigungsfunktion beschrieben (Fig. 3d). Eine ähnliche Gleichung beschreibt die Relation zwischen der Blitzenergie und Lichtanwort in Photorezeptoren. Die Grundlage für die Gleichung und eine molekulare Interpretation von *k* ist durch ein einfaches Modell mit folgenden Annahmen gegeben: Die Bindung eines Resact-Moleküls öffnet alle Ca²⁺-durchlässigen Kanäle innerhalb eines physiologisch definierten Segments des Flagellums. Resact-Moleküle binden entlang des Flagellums entsprechend der Poisson-Verteilung. Die Anzahl von Segmenten ist 1/*k*. Die Wahrscheinlichkeit von 1/*k-*Segmenten, die durch ein oder mehrere Moleküle erregt werden, ist durch die Poisson-Verteilung beschrieben: P(≥1) = 1 - P(0) or 1-*e*^{-*kx*}, wobei P(0) die Wahrscheinlichkeit darstellt, dass eine Zelle kein Resact-Molekül bindet. Dieses Modell beschreibt gut die gezeigte Dosis-Wirkungs-Kurve (Fig. 3d). Es beinhaltet, dass bei geringen Resact-Konzentrationen die Größe der Ca²⁺-Antwort durch die Addition von Einzel-Molekül-Antworten beschrieben werden kann. Die mittlere Anzahl von Segmenten 1/*k* = 10,4 ± 4,2 (n=11) sagt voraus, dass jedes Resact-Molekül ein Segment aktivieren kann, das etwa 5 µm lang ist (bei einer Länge des Flagellums von 50 µm).

### Beispiel 2: Bestimmung des cGMP- und cAMP-Gehaltes in Spermien

Der Zeitverlauf der cGMP- bzw. cAMP-Konzentration nach Stimulierung der Spermien wurde mit Hilfe von *Quenched*-*Flow*-Experimenten bestimmt. Dazu wurde die Messküvette zusammen mit einem *hard-stop*-Ventil und den anderen optischen Komponenten entfernt und gegen ein spezielles Ventil ausgetauscht. Das Ventil ermöglichte es, die aus M3 austretenden Flüssigkeitsgemische in einer Auffangpipette zu sammeln oder sie zu verwerfen. Zusätzlich wurden verschiedene Kombinationen von Reaktionskammern zwischen M1 und M3 eingebaut.

Spermien in ASW mit einer Zelldichte von 0,3 bis 10 x10⁸ Spermien/ml (Spermienstammsuspension) wurden in Spritze 1 gefüllt und mit ASW aus Spritze 2 in M1 gemischt. ASW in Spritze 2 enthielt bestimmte Konzentrationen des SAPs zur Stimulierung der Spermien. Die Spermien wurden weiter in Richtung M3 gedrückt und konnten währenddessen mit dem SAP reagieren. M2 war bedeutungslos, da Spritze 3 nicht gebraucht wurde. In M3 wurden die Spermien durch das Mischen mit HClO₄ (0,5 M Endkonzentration) gequencht d.h., die Spermien wurden unmittelbar getötet, so dass jeder weitere Auf- oder Abbau von cAMP oder cGMP unterdrückt wurde.

**Tabelle 2.**

| Phase | 1 | 2 | 3 |
|---|---|---|---|
| Phasendauer (ms) | 50 | 284 | 454 |
| Spritze 1 (µl) (Spermiensupension) | | 250 | 400 |
| Spritze 2 (µl) (ASW mit SAP) | | 250 | 400 |
| Spritze 3 (µl) (-) | | | |
| Spritze 4 (µl) (HClO₄) | | 250 | 400 |
| Ventil | A | A | B |
| Synchro | On | Off | Off |

**Tab. 2** zeigt ein Beispiel des für die *Quenched-Flow*-Messung verwendeten Spritzenprogramms. Phase 1 diente zum Synchronisieren der Schrittmotoren. Im Verlauf von Phase 2 wurden während 284 ms aus den Spritzen 1, 2 und 4 gleichzeitig jeweils 250 µl durch die Misch- und Reaktionskammern zum Ausgang gedrückt und verworfen (Ventil: A). Phase 2 diente dazu, die von der vorigen Messung übrig gebliebenen Reste der Reaktanden aus Misch- und Reaktionskammern wegzuwaschen. Während Phase 3 wurden jeweils 400 µl aus Spritze 1, 2 und 4 gleichzeitig innerhalb von 454 ms durch die Misch- und Reaktionskammern in die Auffangpipette gedrückt (Ventil: B). In Phase 2 und 3 hatten die angesteuerten Spritzen jeweils eine Ausstoßgeschwindigkeit von 0,845 ml/s. Die Fließgeschwindigkeit der Spermiensuspension aus Spritze 1 verdoppelte sich ab M1 auf 1,690 ml/s durch den gleichstarken Zustrom des SAPhaltigen ASW aus Spritze 2. Die verwendeten Reaktionskammern hatten ein Volumen von 40 µl (I) und 90 µl (II). Zusammen mit dem Mindestvolumen ergab sich ein Gesamtvolumen von 169 µl zwischen M1 und M3. Bei einer Fließgeschwindigkeit von 1,690 ml/s und einem Volumen von 169 µl konnten die Spermien 100 ms mit dem SAP reagieren, bevor die Reaktion in M3 durch Mischen mit HClO₄ gequencht wurde.

**Tabelle 3: Phasendauern in Abhängigkeit von den Reaktionszeiten**

| Reaktionszeit in ms | Phasendauer in ms (Phase 2) | Phasendauer in ms (Phase 3) |
|---|---|---|
| 25 | 72 | 114 |
| 50 | 142 | 227 |
| 100 | 284 | 454 |
| 200 | 568 | 908 |
| 400 | 1136 | 1817 |
| 800 | 2272 | 3634 |
| 1600 | 4544 | 7269 |
| 5000* | 10492 | 3497 |
| 10000* | 20984 | 6995 |

**Tab. 3** gibt die jeweiligen Phasendauern an, die für die verschiedenen Reaktionszeiten erforderlich waren. Bei denen mit * markierten Reaktionszeiten mussten Reaktionskammern mit 1000 µl (I) und 90 µl (II) verwendet werden. Unter dieser Bedingung wurden in Phase 2 jeweils 1200 µl von den Spritzen 1, 2 und 4, anstatt jeweils 250 µl ausgestoßen.

*Quenched-flow*-Messungen wurden mit einer schnellen Mischvorrichtung (SFM-4, Bio-Logic, Frankreich) durchgeführt. Die Temperatur der Proben in den Spritzen wurde bei 17 °C gehalten. Die Spermienlösung wurde schnell (< 1 ms) im Verhältnis 1:1 mit ASW gemischt. Das ASW enthielt die angegebenen Konzentrationen von Resact (Phoenix Pharmaceuticals, USA). Die verschiedenen Stimulationszeiten wurden durch die Flußrate eingestellt (0,11 bis 6,9 ml s⁻¹). Flußraten bis zu 7 ml s⁻¹ ergaben keine mikroskopisch erkennbaren Schäden der Spermienzellen. Ihr Schwimmverhalten nach dem Mischen war normal. Das schnelle Mischen selbst veränderte - ausser bei den beiden größten Flußraten - nicht den cGMP-Gehalt der Zellen, sodass die cGMP-Ruhekonzentration für die Dauer eines Experimentes (bis zu zwei Stunden) größtenteils konstant blieb. Nach der Stimulierung wurde die Spermienlösung schnell mit HClO₄ (1:3 v/v; 0,5 M Endkonzentration) gemischt, um biochemische Reaktionen zu stoppen. Die Proben wurden bis zur weiteren Verwendung tiefgefroren.

Die gefrorenen Proben wurden aufgetaut, kurz gevortext und zum völligen Aufschluss der Spermien fünf Sekunden sonifiziert (Sonifier B-12, Branson Sonic Power Company, Danbury USA, ∼ 90 W). Die Proben wurden durch Zugabe von 400 µl 0,952 M K₃PO₄ (Endkonzentration 0,238 M) neutralisiert. Das KClO₄-Präzipitat und die Zelltrümmer wurden durch Zentrifugation (15.000xg, 15 min, 4°C) sedimentiert. Die cAMP- und cGMP-Konzentrationen wurden jeweils mit 50-200 µl des Überstands mittels Radioimmunoassays (¹²⁵I-cGMP oder ¹²⁵I-cAMP; IBL, Hamburg) nach den Herstellerangaben bestimmt. Kontrollstandards zur Erstellung der Kalibrierungskurven wurden in ASW verdünnt und wie die Spermien mit HClO₄ und K₃PO₄ behandelt. Ein Messpunkt stellt immer den Mittelwert ± der Standardabweichung von drei direkt hintereinander gewonnenen und ausgewerteten Proben dar. Die Standardabweichung von cAMP-Messungen war signifikant größer als die der cGMP-Messungen. Der Grund für diese Variabilität ist unbekannt.

### Schneller Anstieg der intrazellulären cGMP-Konzentration durch Resact

Unstimulierte Spermien enthalten 1,1 ± 0,7 (Durchschnitt ± Standardabweichung) pMole cGMP/10⁸ Zellen (n = 9). Resact stimulierte schnell einen dosisabhängigen großen Anstieg der cGMP-Konzentration (Fig. 1a, b). Die maximale cGMP-Konzentration in Gegenwart von 250 nM Resact war durchschnittlich um den Faktor 30 höher als in unstimulierten Spermien. Bei hohen Resact-Konzentrationen stieg die cGMP-Konzentration innerhalb von 200 ms auf ein Maximum an und sank danach auf kleinere Werten ab.

Die Ruhekonzentration von cAMP betrug 8,3 ± 7,3 pMole cAMP/10⁸ Zellen (n = 10) und war damit deutlich höher als die von cGMP. Die Resact-induzierten cAMP-Antworten hingegen waren deutlich geringer und langsamer als die cGMP-Antworten (Fig. 1c). Die Stimulierung mit 250 nM Resact steigerte die cAMP-Konzentration etwa vierfach. Nach 200 ms hatte sich die cAMP-Konzentration nur unwesentlich verändert, während die cGMF-Konzentration schon auf ihren maximalen Wert angestiegen war (Fig. 1d). Bei geringeren Resact-Konzentrationen (250 pM - 2,5 nM) variierten die Änderungen der cAMP-Konzentration: Sie korrelierten nicht mit den Resact-Konzentrationen und variierten während der Messung (10 s). Nur in zwei von neun Experimenten (eines mit und eines ohne IBMX) zeigte sich ein statistisch signifikanter, stetiger Anstieg der cAMP-Konzentration. Hingegen war der kontinuierliche Anstieg der cGMP-Konzentration mit der jeweils verwendeten Resact-Konzentration abgestuft (Fig. 1b). In Gegenwart von Resact ändert sich demnach die zelluläre cAMP-Konzentration in Spermien nur wenig, die von cGMP hingegen deutlich. Die Vorinkubation der Spermien mit Isobutylmethyxanthin (IBMX), einem Inhibitor von Phosphodiesterasen (PDEs), hat die cGMP-Ruhekonzentration und die anfängliche Geschwindigkeit des cGMP-Anstiegs nicht signifikant verändert. Sie verzögerte bzw. verhinderte jedoch, dass die cGMP-Konzentration wieder von ihrem Maximalwert abnahm (Fig. 1e). Diese Resultate zeigten, dass das Abklingen durch die PDE-Aktivität kontrolliert ist. Im Gegensatz dazu stieg die cAMP-Ruhekonzentration in Gegenwart von IBMX etwa dreifach (23,8 ± 3,5 pMole cAMP/108 Zellen, n = 3) an. Resact (250 nM) steigerte die cAMP-Konzentration zusätzlich etwa um das siebenfache. Bei allen Resact-Konzentrationen stieg die Konzentration der zyklischen Nukleotide stärker an in Gegenwart von IBMX als in Abwesenheit von IBMX (für 250 nM Resact: 90,8 ± 43,3 pMole/10⁸ Zellen (n=4) für cGMP; 178,0 ± 24,6 pMole/10⁸ Zellen (n = 3) für cAMP). Der Resact-induzierte Anstieg der cAMP-Konzentration war in Gegenwart von IBMX signifikant langsamer als in seiner Abwesenheit (Fig. 1f); der langsamere Anstieg deutet auf die Inhibierung der endogenen Adenylatzyklasen (ACs) durch IBMX hin. Zusammenfassend lässt sich sagen, dass die Erstantwort der Spermien aus einem schnellen und dramatischen Anstieg von cGMP besteht. Der verzögerte und geringere Anstieg von cAMP scheint durch verschiedene Downstream-Mechanismen hervorgerufen zu sein.

### Beispiel 3: Caged-Verbindungen und Photolyse

Wir haben neue ([6,7-bis(ethoxycarbonylmethoxy)coumarin-4-yl]methyl-substituierte Formen von cAMP (BECMCM-caged-cAMP) und cGMP (BECMCM-caged-cGMP) (Fig.4a) entwickelt, die den jeweiligen Wirkstoff nach Belichtung mit UV-Licht schnell freigeben. Diese caged-Verbindungen durchdringen schnell die Zellmembranen, ergeben eine relativ große Quantenausbeute (ca. 0,1) und Absorbtivität, und sie sind besonders resistent gegenüber Solvolyse und setzen cAMP oder cGMP innerhalb von etwa 10 ns nach der Belichtung frei. Caged-zyklische Nukleotide wurden in DMSO und caged-Resact ([S-(4,5-dimethoxy-2-nitrobenzyl)Cys⁸]) in 10mM Zitronensäure/NaOH, pH3,0/10mM DTT aufgelöst. Die BECMCM-Verbindungen wurden innerhalb der Zelle durch Esterasen gespalten, und man erhält BCMCM-caged-Derivate. Für die Blitzphotolyse von caged-zyklischen Nukleotiden wurden Spermien mit BECMCM-caged-cGMP oder BECMCM-caged-cAMP (10-100 µM), Fluo4-AM (10 µM) und Pluronic F127 (0,5 %) in ASW für 45-120 min inkubiert. Die zyklischen Nukleotide wurden mittels eines UV-Blitzes von 1,6 ms Dauer aus einer Xe-Blitzlampe (Strobex 278; Chadwick-Helmuth, USA) freigesetzt. Der Blitz wurde durch einen flüssigkeitsgefüllten Lichtleiter und einen zwischengeschalteten Filter (4 mm DUG11, Schott, Deutschland) zur Messküvette geleitet. Die ungeschwächte Blitzenergie an der Küvettenoberfläche betrug 26,8 mJ cm⁻² bei 150 W elektrischem Input. Der belichtete Bereich der Küvette betrug 0,135 cm² (1,5 x 9 mm). Die Energie wurde mit Graufiltern (Quartz) eingestellt.

### Sowohl cGMP als auch cAMP induzieren einen Einstrom von Ca²⁺-Ionen

Resact stimuliert die Synthese von cGMP und cAMP, allerdings mit unterschiedlichen Kinetiken und unterschiedlicher Effizienz. Welcher der beiden Botenstoffe ist für den Ca²⁺-Eintritt verantwortlich? Neue "eingesperrte", sogenannte caged-Formen von cAMP und cGMP (Fig. 4a) ermöglichten uns, diese Frage zu beantworten. In Spermien, die mit caged-cGMP beladen waren, rief die Belichtung mit UV-Blitzen einen ähnlichen Anstieg der intrazellulären Ca²⁺Konzentration hervor wie er mit geringen Resact-Konzentrationen zu beobachten war (Fig. 4b, c und 2b). Sowohl die Amplitude als auch die Verzögerungszeit der Ca²⁺-Signale hingen dabei von der Blitzenergie ab (Fig. 4b, d und f). Die Beziehung zwischen der Signalamplitude und der Blitzenergie (Fig. 4f) ist hinreichend durch die Hill-Gleichung beschrieben. Der Hill-Koeffizient betrug 2,3 ± 0,15 (n = 7), was darauf hindeutet, dass mindestens drei cGMP-Moleküle beim Öffnen des Kanals involviert waren. Der Unterschied zwischen der geringsten Verzögerungszeit des GMP- und Resact-induzierten Ca²⁺-Signals (154 ± 14 ms (n = 4) bzw. 219 ± 37 ms (n = 8)) kann durch die Zeit erklärt werden, die benötigt wird, um cGMP zu synthetisieren. Die Verzögerungen legen nahe, dass Ca²⁺-permeable, durch GMP gesteuerte Ionenkanäle in diesen Vorgang direkt oder indirekt involviert sind.

In Spermien, die mit caged-cAMP beladen waren, rief eine Belichtung mit UV-Blitzen Ca²⁺-Antworten hervor, die sich von denen unterschieden, die durch cGMP hervorgerufen wurden (Fig. 4e). Erstens: Die Zeit bis zur maximalen Amplitude war länger (Fig. 4c), obwohl die Verzögerungszeiten bis dahin größtenteils gleich waren (vgl. Fig. 4b und e). Zweitens: Das Signal blieb auf einem Plateau-Level. Insofern stimmten der zeitliche Ablauf der cAMP- und Resact-induzierten Signale nicht überein (Fig. 4c). Drittens. Die Dosis-Wirkungs-Kurve war weniger steil als die für cGMP und zeigte zudem selbst bei solchen Blitzenergie keine Sättigung, bei denen die cGMP-induzierten Antworten sättigten (Fig. 4f). Die Steilheit und Form der Dosis-Wirkungs-Kurve für cAMP variierte (Fig. 4f): bei geringen Blitzenergien rief cAMP eine größere Ca²⁺-Antwort als cGMP (fünf Experimente) oder eine ähnliche Antwort (drei Experimente) hervor. In drei Experimenten waren die cAMP-induzierten Antworten bei allen Blitzenergien kleiner als die cGMP-induzierten Antworten (Fig.4f). Der Grund für diese Variabilität ist nicht bekannt. Diese Ergebnisse legen nahe, dass cAMP und cGMP einen gemeinsamen Ca²⁺-Signalweg aktivieren, obwohl die Geschwindigkeit und Effizienz dieser Aktivierung durchaus unterschiedlich sein können. Alternativ könnten cAMP und cGMP auch verschiedene Ca²⁺-Signalwege aktivieren.

### Beispiel 4: Analyse der Bewegungsantworten

Die Spermien wurden in ASW/0,2 % Pluronic F127 (1:10⁴- bis 10⁵-Verdünnung von *dry sperm*) suspendiert. Pluronic F127 erhöhte den Anteil frei beweglicher Spermien. Weniger als 40 % der Zellen waren unbeweglich oder an das Glas angeheftet. Ca-freies ASW enthielt nur unbedeutende Mengen an Ca²⁺ ([Ca²⁺] < 10⁻⁵ M); durch die Zugabe von 1 mM EGTA wurde die Ca²⁺-Konzentration nochmals deutlich vermindert ([Ca²⁺] < 10⁻⁹ M); in dem Ca-freien ASW war CaCl₂ durch MgCl₂ ersetzt. Die Spermien wurden durch ein inverses Mikroskop (Zeiss, ICM405) mit Dunkelfeld (Neofluar-Objektive 16/0,40 oder 4.0/0,75) beobachtet. Die Beleuchtung durch eine 100 W Quecksilberlampe wurde durch einen Hitzefilter und einen Langpass-Filter (Schott, KG3 und OG515) gefiltert. Die Beobachtungskammer (Volumen - 16 µl) hatte einen Durchmesser von 20 mm und eine Tiefe von 50 µm. Ihr Boden wurde aus einem Deckglas hergestellt (0,17 mm) und sie wurde mit einem mikroskopischen Objektträger (1 mm) bedeckt. Das Verhalten der Spermien wurde mit einer CCD-Videokamera (Kappa CF 8/1FMC, Bildwiederholungsrate 50 Hz, Belichtungszeit 1 bis 4 ms) und einem Videorekorder (Sony, U-matic VO 5800 PS) aufgezeichnet. Die Bänder wurden Bild für Bild (20 ms-Intervalle) analysiert und die Schwimmkurven manuell auf dem Monitor aufgezeichnet. Originalbilder wurden ausgedruckt (Sony, UP 860 CE) und mit Hilfe elektronischer Datenverarbeitung digitalisiert und überlagert. Dabei diente die Lage der Spermienköpfe als Anhaltspunkt für die Ausrichtung der Bilder.

Resact (2 µl) wurde vorsichtig mit einer Hamilton-Spritze durch einen Schlitz an der Seite der Beobachtungskammer eingespritzt. *Dry sperm* wurde mit ASW/0,2 % Pluronic F 127 1:100 verdünnt, das 100 µM des jeweiligen caged-zyklischen Nukleotids enthielt und für mindestens eine Stunde bei ∼ 12 C° inkubiert. Caged-cAMP und caged-cGMP wurden mit einem UV-Lichtblitz (0,5 ms, UV-Blitzeinheit, TILL Photonics) photolysiert. Der Blitz wurde mittels eines Quartzlichtleiters und eines dichroitischen Spiegels (DCLP 405) durch das Objektiv des Mikroskops auf das Präparat geleitet. Die Beleuchtungsenergie eines ungeschwächten Blitzes betrug etwa 40 mJ cm⁻². Er wurde durch einen Graufilter (Quartz) abgeschwächt. Der Bereich, der durch UV-Licht belichtete wurde, konnte mittels einer rechteckigen Blende (TILL Photonics) eingeschränkt werden. Caged-Resact wurde mit der Spermienlösung mindestens 5 min vor der Belichtung gemischt. Aufgrund der geringen Quantenausbeute von caged-Resact reichte ein Blitz nicht aus, um genügend Resact für eine Antwort freizusetzen. Daher wurde der Blitzvorgang (1 Hz) entweder zehnmal wiederholt (SM Fig. 2) oder der Langpass-Filter aus dem Strahlengang des Beobachtungslichts entfernt (Strahlungsstärke: ∼ 2,5 J cm⁻²s⁻¹ unterhalb 515 nm) (Fig. 7). Die UV-Belichtung in Abwesenheit von caged-Verbindungen löste keine Bewegungsantworten aus. Die Beleuchtungsenergie in der Ebene des Präparates wurde mit einem Radiometer (EG&G 550-1, UV-sensitiver Detektor und Pulsintegrator) und einem Quartzlichtleiter gemessen. Alle Experimente wurden bei 16 °C durchgeführt.

### Schwimmverhalten und "anfängliche" Verhaltensantwort

Spermien schwimmen in einer flachen Beobachtungskammer in Kreisen (Fig. 5a). Mitte der 80er Jahre ist gezeigt worden, dass Resact den Durchmesser der Kreise vergrößert. Diese Beobachtung wurde allerdings nach mehr als zwanzig Sekunden nach der Zugabe von 10 nM Resact gemacht. Daher wird angenommen, dass die beobachteten Schwimmbahnen eher einen adaptierten Zustand der Spermien widerspiegeln als die anfängliche Verhaltensantwort. In unseren Experimenten löste Resact (1 nM) schnelle Änderungen der Bewegung aus, die nicht sofort aufgezeichnet werden konnten, da die Injektion selber Turbulenzen hervorruft. In Gegenwart von IBMX waren die Antworten sehr viel stärker: schon geringe Resact-Konzentration (≥ 10 pM) induzierten ein kräftiges Taumeln ("*tumbling*") der Spermien (nicht zu verwechseln mit dem *tumbling* von Bakterien) (Fig. 5b), das durch Schwimmen in kleinen Kreisen mit einer starken, aber regulären Asymmetrie des Flagellums unterbrochen wurde (Fig. 5c). Dieses Verhalten dauerte mehrere Minuten an. Der Verstärkungseffekt von IBMX deutet darauf hin, dass die Schwimmantworten durch zyklische Nukleotide kontrolliert sind.

Um die Schwierigkeiten bei der Aufzeichnung der frühen Bewegungsantworten zu überwinden, haben wir eine caged-Form von Resact entwickelt, bei der der Cystein-Rest an Position 8 (Cys8) kovalent modifiziert wurde. Modifikationen am Cys8 beeinträchtigten nachhaltig die Fähigkeit von Resact, die Konzentration zyklischer Nukleotide in Spermien zu erhöhen. Wir verglichen die Fähigkeit von caged-Resact mit der von unmodifiziertem Resact, indem wir in *Stopped-Flow-*Experimenten die entsprechenden Ca²⁺-Antworten gemessen haben. Caged-Resact (4 nM) induzierte einen Anstieg der intrazellulären Ca²⁺-Konzentration, der in seiner Größe und seiner Gestalt dem durch 1,25 pM Resact induzierten Anstieg ähnelte (Fig. 6a). Die Dosis-Wirkungs-Kurven (Fig. 6b) zeigen, dass caged-Resact etwa 3.000mal weniger effektiv ist als unmodifiziertes Resact. Nachdem das Ca²⁺-Signal, das durch caged-Resact ausgelöst wurde, abgeklungen war, konnte mit einem UV-Blitz ein zweites Ca²⁺-Signal ausgelöst werden (Fig. 6c). Durch einen Vergleich der Amplituden der Ca²⁺-Signale, die durch normales Resact bzw. durch Photolyse von caged-Resact ausgelöst wurden, ließ sich abschätzen, dass jeder Blitz etwa 100 pM Resact aus 0,5 µM caged-Resact freisetzen konnte, also etwa 0,02 %.

In Gegenwart von caged-Resact (1 µM) schwammen Spermien in normalen Kreisen (Durchmesser: 68 ± 21 µm; n = 83), die etwas größer waren als die von Kontrollspermien. Nach Belichtung eines Bereichs der Beobachtungskammer, die etwa 10 µM caged-Resact enthielt, sammelten sich die Spermien in diesem Bereich innerhalb von etwa 20 s an (Fig. 10a, b). Dies bewies, dass Spermien auf einen sprunghaften Anstieg der Resact-Konzentration auf einer Hintergrundskonzentration von etwa 3 nM Resact noch ansprachen. Diese Anpassung der Sensitivität, genannt Adaptation, ist eine wichtige Voraussetzung, um einen Gradienten eines Lockstoffes zu entdecken.

Resact-induzierte Bewegungsantworten wurden untersucht, indem die Schwimmbahnen einzelner Spermien verfolgt wurden. Nach der Photolyse von caged-Resact schwammen die Spermien zunächst (mehrere 100 ms lang) weiter entlang ihres ursprünglichen Weges (Fig. 7). Danach setzten Wendereaktionen ("*turns*")- oder Taumelbewegungen ("tumbling") ein, die von einer transienten Änderung des Flagellen-Schlagmusters begleitet wurden. Dieses Schlagmuster war charakterisiert durch ein starkes Biegen ("*bending*") des proximalen Bereichs und ein "Stretchen" des distalen Bereichs des Flagellums ("hakenförmige" Gestalt). Oftmals folgte diesem Verhalten eine Periode eines nahezu geradlinigen Schwimmverhaltens. Die Antworten wurden bei Konzentration von 100 pM und mehr caged-Resact beobachtet. Wenn man annimmt, dass nur ein Bruchteil des caged-Resacts photolysiert wurde, so waren die Spermien also in der Lage, auf Konzentrationen im Pikomolarbereich zu antworten, d.h. in einem Konzentrationsbereich von Resact, der einen Ca²⁺-Einstrom in die Spermien auslöst. Der Anteil der antwortenden Zellen und die Reaktionszeit, d.h. die Zeit, die zwischen der Belichtung und der ersten Wende verstrich, war abhängig von der Konzentration an caged-Resact (Fig. 7).

Um zu testen, ob cGMP die Bewegungsantwort hervorruft, haben wir Spermien mit caged-cGMP beladen. Ebenso wie mit caged-Resact, induzierte UV-Belichtung einen transienten Anstieg der Flagellen-Asymmetrie, die zunächst eine Wende oder ein Taumeln der Spermien bewirkte und dann in ein geradliniges Schwimmen überging (Fig. 8a-d). Die ursprüngliche Verhaltensweise wurde innerhalb von wenigen Sekunden wieder hergestellt. Starke Blitze bewirkten oft eine Abfolge von 2 bis 4 *turns,* die durch Intervalle (150-250ms) geradlinigen Schwimmens unterbrochen wurden. Der Anteil an antwortenden Zellen und die Reaktionszeit hing von der Blitzenergie ab (Fig. 8f). Die Antworten auf cAMP waren qualitativ ähnlich zu denen, die durch cGMP hervorgerufen wurden; jedoch war für sämtliche Blitzenergien die Reaktionszeit der Spermien signifikant länger (p < 0,005). Die zwei schwächsten Blitze lösten überhaupt keine cAMP-Antwort aus.

In einem Medium, das eine äußerst geringe Ca²⁺-Konzentration (Ca²⁺ < 10⁻⁹ M) hatte, schwammen die Spermien normal. Nach Photolyse von caged-Resact (1 µM) wurden jedoch weder eine Ansammlung der Spermien noch Wendereaktionen beobachtet. Stattdessen wurde der Kreisdurchmesser stetig größer mit einer Latenzzeit von 1076 ± 391 ms (n = 42). Ähnlich dazu wurden auch mit caged-cGMP keine Blitz-induzierten Wendereaktionen beobachtet und nach einer Latenzzeit von 299 ± 67 ms (n = 19) stieg der Kreisdurchmesser stetig an (Fig. 8e). Dieses Ergebnis deutet darauf hin, dass die intrazelluläre Ca²⁺-Konzentration unter diesen Bedingungen abnimmt. Alternativ könnte cGMP neben der Aktivierung der Ca²⁺-Leitfähigkeit weitere zelluläre Reaktionen kontrollieren.

Zusammenfassend lässt sich sagen, dass die Reaktionszeit für die Resact-induzierte Bewegungsantwort (350-1000 ms) geringfügig länger war als die Verzögerung (200-600 ms), aber kürzer als die Zeit bis zum Maximalwert (1-2 s) des Resact-induzierten Ca²⁺-Signals. Darüber hinaus stimmt die kürzeste Reaktionszeit (∼ 240 ms), die mit gecagtem cGMP erhalten wurde, gut mit der kürzesten Verzögerung (∼ 150 ms) des cGMP-induzierten Ca²⁺-Signals überein. Diese Ergebnisse legen nahe, dass die Bewegungsantworten durch einen cGMPvermittelten Anstieg von intrazellulärem Ca²⁺ kontrolliert wurden. Weil Resact einen stärkeren und schnelleren Anstieg der cGMP-Konzentration auslöst und weil cGMP schneller das Schwimmverhalten der Spermien beeinflußt, muß man schließen, dass cAMP die primäre Bewegungsantwort nicht kontrolliert. Während die Wendereaktion vermutlich auf einem moderaten Anstieg der intrazellulären Ca²⁺-Konzentration beruhen, deutet das Taumeln hingegen auf eine starke Erhöhung der intrazellulären Ca²⁺-KonzentrationCa²⁺ hin. Das Taumeln erinnert dabei an die Ca²⁺-abhängige Hyperaktivierung von Säugerspermien.

## Patentansprüche

1. Verfahren zum Nachweis von Änderungen von zellulären Botenstoffen in Spermien in einer *Stopped-Flow*-Messvorrichtung, enthaltend die folgenden Verfahrensschritte:
a) Mischen von Spermien und Wirkstoff, wobei der Wirkstoff mit einer chemischen Substanz kovalent verbunden ist,
b) Freisetzen des Wirkstoffs durch Photolyse mittels eines UV-Blitzes und
c) Nachweisen der Änderung mittels Stopped- oder Quenched-Flow-Messung, wobei zwischen Schritt a) und Schritt c) nicht mehr als etwa 5 ms bis 1000 ms liegen.

2. Verfahren nach Anspruch 1, wobei der Botenstoff cAMP, cGMP, IP3, Ca²⁺-Gehalt, pH-Wert, zyklische ADP-Ribose oder das Membranpotential ist.

3. Verfahren nach Anspruch 1 oder 2, wobei der Nachweis mit fluoreszierenden oder lumineszierenden Indikatoren durchgeführt wird.

4. Verfahren nach Anspruch 1 oder 2, wobei der Nachweis der Änderung des intrazellulären pH-Wertes mit BCECF durchgeführt wird.

5. Verfahren nach Anspruch 1 oder 2, wobei der Nachweis der Änderung der intrazellulären Ca²⁺-Konzentration mit Fluo4 durchgeführt wird.

6. Verfahren nach Anspruch 1 oder 2, wobei der Nachweis der Änderung der intrazellulären cAMP- oder cGMP-Konzentration mittels eines radioaktiven, Lumineszenz- oder Fluoreszenz-optischen hnmunnachweises durchgeführt wird.

7. Verfahren nach einem der vorherigen Ansprüche, wobei die Spermien Wirbeltierspermien sind.

8. Verfahren nach Anspruch 7, wobei die Spermien vom Schwein, Rind, Lamm, Pferd, Geflügel, Ratte, Maus oder Menschen stammen.

9. Verfahren nach einem der vorherigen Ansprüche, wobei der Wirkstoff eine Körperflüssigkeit, ein Zellextrakt, eine einzelne Zelle oder eine synthetisch hergestellte Substanz ist.

10. Verfahren nach Anspruch 9, wobei die Körperflüssigkeit eine intrauterine oder intratubare Körperflüssigkeit ist.

11. Verfahren nach einem der vorherigen Ansprüche, wobei der mit einer chemischen Substanz kovalent verbundene Wirkstoff cAMP, kovalent verbunden mit [6,7-bis(ethoxycarbonylmethoxy)coumarin-4-yl]methyl oder cGMP, kovalent verbunden mit [6,7-bis(ethoxycarbonylmethoxy)coumarin-4-yl]methyl oder Resact, kovalent verbunden mit [S-(4,5-dimethoxy-2-nitrobenzyl)Cys⁸] ist.

12. Resact, kovalent verbunden mit [S-(4,5-dimethoxy-2-nitrobenzyl)Cys⁸].

13. Verwendung des Verfahrens nach einem der Ansprüche 1-11 zur Identifizierung oder Charakterisierung von Wirkstoffen, die den Befruchtungsvorgang beeinflussen, insbesondere die Fertilität von Spermien steigern und/oder kontrazeptiv wirken.

14. Verwendung nach Anspruch 13 zum Durchmustern von Wirkstoffbibliotheken.

15. Verwendung des Verfahrens nach einem der Ansprüche 1-11 zur Entwicklung oder Charakterisierung von Substanzen zur Bestimmung der Fertilität von Spermien und Eizellen.

## Claims

1. Method for detecting alterations of cellular messengers in sperms with a *stopped-flow* measurement apparatus, comprising the following steps:
a) mixing of sperms and active agent, wherein the active agent is covalently linked to a chemical substance,
b) releasing of the active agent by means of UV flash photolysis and
c) detecting the alteration by means of stopped- or quenched-flow measurement, wherein between step a) and step c) 5 ms to 1000 ms are not exceeded.

2. Method of claim 1, wherein the messenger is cAMP, cGMP, IP3, Ca²⁺ concentration, pH-value, cyclic ADP-ribose or membrane potential.

3. Method of claim 1 or 2, wherein the detection is carried out by means of fluorescent or luminescent indicators.

4. Method of claim 1 or 2, wherein the detection of alteration of the intracellular pH-value is carried out by means of BCECF.

5. Method of claim 1 or 2, wherein the detection of alteration of the intracellular Ca²⁺ concentration is carried out by means of Fluo4.

6. Method of claim 1 or 2, wherein the detection of alteration of the intracellular cAMP or cGMP concentration is carried out by means of a radioactive, luminescent- or fluorescent-optical immunodetection.

7. Method of any of the preceding claims, wherein the sperms are vertebrate sperms.

8. Method of claim 7, wherein the sperms are derived from pig, cow, sheep, horse, poultry, rat, mouse, or human.

9. Method of any of the preceding claims, wherein the active agent is a body fluid, a cell extract, a single cell, or a synthetically produced substance.

10. Method of claim 9, wherein the body fluid is an intrauterine or intratubular body fluid.

11. Method of any of the preceding claims, wherein the active agent, covalently linked to a chemical substance, is cAMP, covalently linked to [6,7-bis(ethoxycarbonylmethoxy)coumarin-4-yl]methyl, or cGMP, covalently linked to [6,7-bis(ethoxycarbonylmethoxy)coumarin-4-yl]methyl, or Resact, covalently linked to [S-(4,5-dimethoxy-2-nitrobenzyl)Cys⁸].

12. Resact, covalently linked to [S-(4,5-dimethoxy-2-nitrobenzyl)Cys⁸].

13. Use of a method of any of claims 1 to 11 for identifying or characterizing active agents affecting the process of fertilization, in particular increasing fertility of sperms and/or acting contraceptively.

14. Use according to claim 13 for screening active agent libraries.

15. Use of a method of any of claims 1 to 11 for developing or characterizing substances for determining fertility of sperms or ovum cells.

## Revendications

1. Procédé de détection des modifications des messagers cellulaires dans les spermatozoïdes dans un dispositif de mesure à flux stoppé, comprenant les étapes suivantes de procédé :
a) le mélange des spermatozoïdes et d'un principe actif, le principe actif étant lié à une substance chimique de manière covalente,
b) la libération du principe actif par photolyse au moyen d'un éclair d'UV et
c) la détection de la modification au moyen de la mesure à flux stoppé ou refroidi, où il n'y pas plus d'environ 5 ms à 1000 ms entre l'étape a) et l'étape c).

2. Procédé selon la revendication 1, dans lequel le messager est l'AMPc, le GMPc, l'IP3, la teneur en Ca²⁺, le pH, l'ADP-ribose cyclique ou le potentiel membranaire.

3. Procédé selon la revendication 1 ou 2, dans lequel la détection est réalisée avec des indicateurs fluorescents ou luminescents.

4. Procédé selon la revendication 1 ou 2, dans lequel la détection de la modification du pH intracellulaire est réalisée avec BCECF.

5. Procédé selon la revendication 1 ou 2, dans lequel la détection de la modification de la concentration du Ca²⁺ intracellulaire est réalisée avec Fluo4.

6. Procédé selon la revendication 1 ou 2, dans lequel la détection de la modification de la concentration en AMPc ou en GMPc intracellulaire est réalisée au moyen d'une détection immunitaire radioactive, optique par luminescence ou fluorescence.

7. Procédé selon l'une des revendications précédentes, dans lequel les spermatozoïdes sont des spermatozoïdes de vertébré.

8. Procédé selon la revendication 7, dans lequel les spermatozoïdes proviennent de porcs, de boeufs, d'agneaux, de chevaux, de volailles, de rats, de souris ou de l'homme.

9. Procédé selon l'une des revendications précédentes, dans lequel le principe actif est un fluide corporel, un extrait cellulaire, une cellule individuelle ou une substance produite synthétiquement.

10. Procédé selon la revendication 9, dans lequel le fluide corporel est un fluide corporel intra-utérin ou intratubaire.

11. Procédé selon l'une des revendications précédentes, dans lequel le principe actif lié de manière covalente à une substance chimique est l'AMPc, lié de manière covalente au [6,7-bis(éthoxycarbonylméthoxy)coumarine-4-yl]-méthyle ou le GMPc, lié de manière covalente au [6,7-bis(éthoxycarbonylméthoxy)coumarine-4-yl]-méthyle ou Resact, liée de manière covalente à la [S-(4,5-diméthoxy-2-nitrobenzyl)Cys⁸].

12. Resact, liée de manière covalente à la [S-(4,5-diméthoxy-2-nitrobenzyl)Cys⁸].

13. Utilisation du procédé selon l'une des revendications 1 à 11 pour identifier ou caractériser des principes actifs, qui influencent le processus de fécondation, augmentent notamment la fertilité des spermatozoïdes et/ou ont une action contraceptive.

14. Utilisation selon la revendication 13 pour cribler des bibliothèques de principes actifs.

15. Utilisation du procédé selon une des revendications 1 à 11 pour le développement ou la caractérisation de substances pour la détermination de la fertilité des spermatozoïdes et des ovules.
